# EUROPÄISCHE PATENTSCHRIFT

(11) **EP 3 793 495 B1**
(45) Veröffentlichungstag und Bekanntmachung des Hinweises auf die Patenterteilung: **13.12.2023**
(21) Anmeldenummer: 19726606.7
(22) Anmeldetag: 17.05.2019
(51) Int. Cl.: A61F 13/49, A61F 13/491, A61F 13/494, A61F 13/496

(54) **INKONTINENZARTIKEL IN HÖSCHENFORM**
INCONTINENCE ARTICLE IN THE FORM OF BRIEFS
ARTICLE D'INCONTINENCE SOUS FORME DE CULOTTE

(30) Priorität: 18.05.2018 DE 102018112119
(43) Veröffentlichungstag der Anmeldung: 24.03.2021
(73) Patentinhaber: Paul Hartmann AG, 89522 Heidenheim (DE)
(72) Erfinder: BUCH, Tamara, 89077 Ulm (DE); EILERS, Joerg, 89179 Beimerstetten (DE); KESSELMEIER, Ruediger, 89233 Neu-Ulm (DE); BEYRLE, Andreas, 89564 Nattheim (DE); ROHRBACHER, Agnes, 89520 Heidenheim (DE); SCHMIDT, Ann-Cathrin, 06667 Weissenfels (DE)
(74) Vertreter: DREISS Patentanwälte PartG mbB
(86) Internationale Anmeldenummer: PCT/EP2019/062873
(87) Internationale Veröffentlichungsnummer: WO 2019/219943

(56) Entgegenhaltungen:
- WO-A1-00/47152
- DE-A1-102005 030 182

## Beschreibung

Die Erfindung betrifft einen Inkontinenzartikel in Höschenform, also einen "pull-up"-Artikel, für die Aufnahme von Körperausscheidungen für die bevorzugte Verwendung durch Männer, mit einem vorderen Bauchabschnitt und einem hinteren Rückenabschnitt, die in einer Längsrichtung voneinander beabstandet sind und die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in der Längsrichtung zwischen Bauchabschnitt und Rückenabschnitt erstreckt und an den Bauchabschnitt und an den Rückenabschnitt in einem jeweiligen Überlappungsbereich unlösbar angefügt ist, wobei der Schrittabschnitt, der Bauchabschnitt und der Rückenabschnitt gemeinsam jeweilige Beinöffnungen des Inkontinenzartikels begrenzen, wobei der Schrittabschnitt beidseits eine seitliche Auslaufsperre bildende und beidseits entlang der Längserstreckung des Absorptionskörpers verlaufende Cuffelemente aufweist, die zumindest entlang einer Cuffsockellinie an der körperzugewandten Seite des Artikels festgelegt sind und einen unbefestigten freien Längsrand, der zumindest abschnittsweise in Längsrichtung elastifiziert ist, aufweisen, mit dem sich die Cuffelemente von der körperzugewandten Seite des Artikels emporerheben können und so die jeweilige seitliche Auslaufsperre bilden und wobei der Absorptionskörper so angeordnet ist, dass eine Quermittellinie des Absorptionskörpers zwischen der Quermittelachse des Inkontinenzartikels und einer Hüftöffnungskante des Bauchabschnitts angeordnet ist.

DE102005030182 offenbart ein wegwerfbares absorbierendes Inkontinenzprodukt.

Aus der WO 2005/044169 A1 ist ein wegwerfbares absorbierendes Inkontinenzprodukt für Männer bekannt, das einen nach vorne verschobenen Absorptionskörper aufweist, um eine Anpassung an die männliche Anatomie und den Ort des Flüssigkeitsanfalls bereit zu stellen.

Dieser Inkontinenzartikel in Höschenform soll als genderspezifisches Produkt für den männlichen Träger weiterentwickelt werden. Der Inkontinenzartikel ist insbesondere ein Erwachseneninkontinenzprodukt. Der Inkontinenzartikel soll gegenüber dem Bekannten noch besser an die männliche Anatomie angepasst werden.

Diese Aufgabe wird erfindungsgemäß durch einen Inkontinenzartikel mit den Merkmalen des Anspruchs 1 gelöst.

Es wurde also erfindungsgemäß von herkömmlichen bei derartigen Inkontinenzartikeln typischerweise eingesetzten durchgehend über die gesamte Länge elastifizierten Cuffsystemen Abstand genommen. Diese vorbekannten Cuffsysteme waren typischerweise so ausgebildet, dass ein jeweiliges Cuffelement in der Regel im Bereich seines freien emporerhebbaren Längsrands elastifiziert ist. Im Ergebnis führt der Verzicht auf eine Elastifizierung des freien Cufflängsrandes im vorderen Bereich zu einem angenehmen Tragegefühl. Elastifizierungsmittel weisen häufig das Problem auf, dass sie zu einer Reizung oder Irritation der Haut führen und so den Tragekomfort beeinträchtigen können. Insbesondere besteht ein Problem bei elastifizierten Bereichen, wenn diese in sensiblen zugleich mit einer Behaarung versehenen Regionen des Anwenders zum Einsatz kommen. Es ist daher der Gedanke der Erfindung, einen geschlechtsspezifischen Inkontinenzartikel bereitzustellen, der insbesondere in dem für männliche Nutzer kritischen vorderen Bereich bezüglich der Cuffelemente frei von elastischen Mitteln in den freien Längsrändern der Cuffelemente ist. Auf diese Weise kann zudem in diesem Bereich eine Überlagerung der elastischen Mittel verhindert werden, sofern wie üblich auch eine Elastifizierung der Beinausschnitte vorgenommen wird. Die bevorzugt eingesetzten faden- oder bandförmigen Elastifizierungsmittel bewirken eine Raffung oder Rüschung von mit ihnen verbundenen Materialien, wenn sie im vorgespannten Zustand mit den Materialien verbunden werden. Dies führt im relaxierten Zustand zu einer Materialhäufung, die als unangenehm empfunden werden kann. Die gerafften oder gerüschten Bereiche können gegen die Hautoberfläche reiben, was zu den erwähnten Hautirritationen führen kann. Bei einer Überlappung von mehreren elastischen Komponenten wird dieser Effekt und damit die Wahrscheinlichkeit des Auftretens von Hauteffekten noch verstärkt.

Auf der anderen Seite ist eine Cuffelastifizierung wünschenswert, um ein sicheres Emporerheben und Aufstellen der Cuffelemente zu gewährleisten, gerade bei größeren Absorptionskörpern, wie sie bei für Männer geeigneten Inkontinenzartikeln häufig vorgesehen sind, und damit ihre Funktion als seitliche Auslaufsperre.

Dabei hat es sich als vorteilhaft erwiesen, dass der jeweilige unbefestigte freie Längsrand der beiden Cuffelemente zum vorderen Bauchabschnitt hin einen nicht elastifizierten freien Längsrandendabschnitt aufweist, dessen Längserstreckung C1 wenigstens 12%, insbesondere mindestens 15%, weiter insbesondere mindestens 20% und insbesondere höchstens 45%, weiter insbesondere höchstens 40%, weiter insbesondere höchstens 35% einer Längserstreckung C2 des unbefestigten freien Längsrands der Cuffelemente in der sich von der Quermittelachse des Inkontinenzartikels zum Bauchabschnitt erstreckenden Produkthälfte beträgt.

Der freie Längsrand ist dabei der der Cuffsockellinie gegenüberliegende Endbereich eines Cuffelements in proximaler/distaler Anordnung.

Eine Elastifizierung erfolgt dabei entweder über eine flächenhafte Elastifizierung der Cuffelemente, indem die Cuffelemente in Längsrichtung elastisch dehnbare Flächenmaterialien umfassen. Insbesondere kann vorgesehen sein, dass in die Cuffelemente im Bereich ihres freien emporerhebbaren Längsrandes im Wesentlichen fadenförmige Elastifizierungsmittel eingesetzt werden oder streifenförmige Elastifizierungsmittel.

Im nicht elastifizierten Längsrandendabschnitt erstreckt sich der Verzicht eines Elastifizierungsmittels erfindungsgemäß zumindest im Bereich des freien Längsrandes. Weiter vorteilhaft ist ausgehend vom freien Längsrand innerhalb einer Quererstreckung zur Cuffsockellinie zumindest innerhalb des oberen Viertels, weiter vorteilhaft des oberen Drittels, weiter vorteilhaft der oberen Hälfte, weiter vorteilhaft innerhalb der gesamten Querstreckung bis zur Cuffsockellinie keine Elastifizierung der Cuffelemente vorgesehen.

Insbesondere bevorzugt ist vorgeschlagen, dass die Längserstreckung C1 des nicht elastifizierten Längsrandendabschnittes der Cuffelemente wenigstens 8%, insbesondere wenigstens 10% und insbesondere wenigstens 12% sowie höchstens 30%, insbesondere höchstens 28%, insbesondere höchstens 25% und insbesondere höchstens 23% einer Gesamtlängserstreckung C3 des unbefestigten freien Längsrands der Cuffelemente beträgt.

Nach einer bevorzugten Ausgestaltung der Erfindung kann dabei vorgesehen sein, dass das Verhältnis einer Länge S1 des Absorptionskörpers vor der Quermittelachse des Inkontinenzartikels in Richtung Bauchabschnitt zu einer Länge S2 des Absorptionskörpers hinter der Quermittelachse des Inkontinenzartikels in Richtung Rückenabschnitt mindestens 1,2, vorzugsweise mindestens 1,3 und insbesondere höchstens 2,0, insbesondere höchstens 1,8 und insbesondere höchstens 1,6 beträgt. Im Gegenzug dazu beträgt das Verhältnis bei einem bevorzugt für Frauen konzipierten Produkt im Wesentlichen 1. Durch die Vorverlagerung des Absorptionskörpers kann die männliche Anatomie noch besser abgebildet werden. Dabei wird die Vorverlagerung durch den Wunsch, auch im tiefsten Punkt des Inkontinenzartikels ausreichend Absorptionskapazität bereit zu stellen, beschränkt, so dass sich der genannte Bereich als besonders vorteilhaft herausgestellt hat.

Die Quermittelachse des Inkontinenzartikels bestimmt sich dabei durch die Halbierung der Länge des Inkontinenzartikels. Die Quermittellinie des Absorptionskörpers bestimmt sich durch die Halbierung der Länge des Absorptionskörpers. Die Längsmittelachse des Inkontinenzartikels bzw. des Absorptionskörpers ergibt sich aus der jeweiligen Halbierung in Längsrichtung.

Dabei ist es weiterhin insbesondere vorteilhaft, wenn das Verhältnis einer Länge des Absorptionskörpers S1 vor der Quermittelachse in Richtung Bauchabschnitt zu einer Länge L1 des Inkontinenzartikels ausgehend von der Quermittelachse bis zu einer die Hüftöffnung begrenzenden Vorderkante des Bauchabschnitts mindestens 0,6, insbesondere mindestens 0,7 beträgt, um die oben genannten Vorteilen zu erreichen.

Weiterhin ist es bevorzugt, dass der elastifizierte Abschnitt des freien Längsrands der Cuffelemente in seiner Längserstreckung von der Quermittelachse des Inkontinenzartikels in Richtung Bauchabschnitt um höchstens 10%, insbesondere höchstens 8% und insbesondere höchstens 6% größer ist bezogen auf die Längserstreckung von der Quermittelachse des Inkontinenzartikels in Richtung Rückenabschnitt, insbesondere dass der elastifizierte Abschnitt des freien Längsrands der Cuffelemente in seiner Längserstreckung im Wesentlichen symmetrisch in Längsrichtung zur Quermittelachse angeordnet ist. Durch diese weitgehend symmetrische Anordnung der elastifizierten Bereiche kann eine hinreichende Taschenbildung und ein ausreichendes Emporerheben und Aufrichten der Cuffelemente im Schrittbereich sichergestellt werden und gleichzeitig die Nachteile der Elastifizierung insbesondere bei einem geschlechtsspezifischen Einsatz minimiert werden.

Dabei kann vorgesehen sein, dass die Cuffelemente an die freien nicht elastifizierten Längsrandendabschnitte in Längsrichtung anschließende fixierte Endrandabschnitte aufweisen, mit denen die Cuffelemente an der körperzugewandten Seite des Inkontinenzartikels festgelegt sind, wobei die Cuffelemente einwärts ihrer Cuffsockellinie zuliegen kommen und insbesondere in den fixierten Endrandabschnitten gefaltet eingebracht sind, insbesondere derart, dass der freie Längsrand auswärts weist. Diese Anordnung kann durch eine C- oder Z-Faltung der Cuffelemente erreicht werden, die zu einer guten Taschenbildung der Cuffelemente im Bereich unmittelbar benachbart der fixierten Endrandabschnitte beiträgt.

Weiter vorzugsweise beträgt das Verhältnis eines Abstands E zwischen den nach der Fixierung im fixierten Endrandabschnitt verbliebenen freien Kanten der Cuffelemente zum maximalen Abstand D der Cuffsockellinien mindestens 0,3, weiter insbesondere mindestens 0,4, weiter insbesondere höchstens 0,7, weiter insbesondere höchstens 0,6, weiter insbesondere höchstens 0,5. Als maximaler Abstand D wird hierbei ausgehend vom fixierten Endrandabschnitt und dabei beidseits in Längsrichtung dem Verlauf der Cuffsockellinien in Richtung Quermittelachse folgend dann der dem fixierten Endrandabschnitt dazu nächstgelegene maximale Abstand der Cuffsockellinien herangezogen. Der Abstand zwischen den nach der Fixierung verbliebenen freien Kanten der Cuffelemente, Abstand E - vermessen im Übergang vom nicht elastifizierten Längsrandendabschnitt zum fixierten Endrandabschnitt beträgt insbesondere mindestens 40 mm, weiter vorzugsweise mindestens 50 mm, weiter insbesondere höchstens 75 mm, weiter vorzugsweise höchstens 70 mm.

Mit diesen Abständen und Bemessungen wird im vorderen Bereich eine der männlichen Anatomie angepasste Taschenbildung und auch Platzierung der männlichen Genitalien beim Anziehen des Inkontinenzartikels unterstützt.

Insbesondere bevorzugt weisen im Übergang des elastifizierten freien Längsrand, also dessen vorderes Ende, zum nicht elastifizierten freien Längsrandendabschnitt die Cuffelemente jeweils eine Höhe und die Cuffsockellinien einen Abstand zueinander auf, wobei das Verhältnis der Höhe des Cuffelements zum Abstand der Cuffsockellinien mindestens 0,25, insbesondere mindestens 0,30, weiter insbesondere mindestens 0,35, weiter insbesondere höchstens 0,50, weiter insbesondere höchstens 0,45, weiter insbesondere höchstens 0,40 beträgt. Hierdurch kann ein optimales Verhältnis des Abstandes der Cuffsockellinien zur Höhe der Cuffelemente in diesem Übergangspunkt von elastifizierten in den nichtelastifzierten Bereich erzielt werden, um ein Aufspannen und eine Taschenbildung der Cuffelemente als Auslaufsperre zu erreichen.

Die Höhe der Cuffelemente wird als Quererstreckung des von der körperzugewandten Seite des Artikels emporerhebbaren Bereichs des jeweiligen Cuffelements ausgehend von der Cuffsockellinie bis zum freien Längsrand vermessen.

Unter Cuffsockellinie wird die Verbindungsstelle zwischen dem emporerhebbaren Bereich des Cuffelements und einer körperzugewandten Lage des Schrittabschnitts verstanden.

Die Cuffsockellinie ergibt sich vorzugsweise aus Ultraschallschweißbereichen und/oder adhäsiven Verbindungsbereichen. Vorzugsweise sind die Cuffsockellinien der beiden Cuffelemente in einem Abstand von 120 - 170 mm, insbesondere von 130 - 160 mm, insbesondere 130 - 150 mm voneinander beabstandet.

Insbesondere vorteilhaft beträgt die maximale Höhe der Cuffelemente, also die Cuffhöhe wenigstens 35 mm, insbesondere wenigstens 40 mm, insbesondere wenigstens 45 mm, insbesondere wenigstens 50 mm und insbesondere höchstens 65 mm, insbesondere höchstens 60 mm, insbesondere höchstens 55 mm, wobei die maximale Cuffhöhe als jeweilige Quererstreckung des von der körperzugewandten Seite des Artikels emporerhebbaren Bereichs des jeweiligen Cuffelements bestimmt wird.

Vorzugsweise ist vorgesehen, dass im Übergang eines vorderen Endes des elastifizierten Abschnitts des freien Längsrandes zum nicht elastifizierten freien Längsrandendabschnitt die Cuffsockellinie innerhalb eines Randes des Absorptionskörpers verläuft, also oberhalb des Absorptionskörpers angeordnet ist.

Diese Anordnung unterstützt vorteilhaft die Taschenbildung im vorderen Bereich.

Es kann insbesondere vorgesehen sein, dass das Verhältnis einer maximalen Höhe der Cuffelemente ausgehend von der Cuffsockellinie zum freien Längsrand zu einer kleinsten Breite K des Absorptionskörpers mindestens 0,25, insbesondere mindestens 0,30, insbesondere mindestens 0,35 und insbesondere mindestens 0,40 sowie insbesondere höchstens 0,60, weiter insbesondere höchstens 0,55 beträgt, wobei die kleinste Breite K des Absorptionskörpers als Querstreckung an einer Stelle des Absorptionskörpers bestimmt wird, die in der Längsrichtung zwischen dem Bauchabschnitt und dem Rückenabschnitt liegt. Bevorzugt wird die Vermessung der kleinsten Breite an der Quermittelachse des Inkontinenzartikels durchgeführt. Hierdurch wird vorteilhaft erreicht, dass das Aufstellen der Cuffelemente erleichtert wird und ein Zusammenfallen der Cuffelemente auf dem Absorptionskörper verhindert wird, was zu einem Verlust der Flüssigkeitssperrwirkung führen würde.

Insbesondere kann ein Abstand M zwischen einer Längskante des Absorptionskörpers an dessen kleinster Breite K und der davon auswärts angeordneten Cuffsockellinie vorgesehen sein, wobei das Verhältnis zwischen dem Abstand M und der kleinsten Breite K des Absorptionskörpers mindestens 0,10, insbesondere mindestens 0,12, insbesondere mindestens 0,14, weiter insbesondere höchstens 0,25, weiter insbesondere höchstens 0,22, weiter insbesondere höchstens 0,20 beträgt, wobei der Abstand M und die minimale Breite des Absorptionskörpers als Quererstreckung an einer Stelle des Absorptionskörpers bestimmt wird, die in der Längsrichtung zwischen dem Bauchabschnitt und dem Rückenabschnitt liegt. Bevorzugt wird die Vermessung der kleinsten Breite an der Quermittelachse des Inkontinenzartikels durchgeführt. Durch die Taillierung des Absorptionskörpers bei vorzugsweise geradem Verlauf der Cuffsockellinie wird erreicht, dass sich die Cuffelemente noch besser aufstellen können. Daher kann es wünschenswert sein, dass im Wesentlichen eine gerade, insbesondere zur Längsmittelachse parallel verlaufende Cuffsockellinie im mittleren Bereich, insbesondere in Längsrichtung innerhalb der schrittseitigen Enden der Seitennahtbereiche vorgesehen ist.

Der Absorptionskörper kann tailliert, d.h. im Bereich der Quermittellinie des Absorptionskörpers mit geringerer Breite ausgebildet sein, so dass er im Wesentlichen eine sanduhrförmige Konfiguration annimmt.

Insbesondere bevorzugt weist der Absorptionskörper in einem zum Bauchabschnitt gerichteten vorderen Bereich eine größere Breite auf als im zum Rückenabschnitt gerichteten hinteren Bereich. Dies trägt vorteilhaft zu einer an die männliche Anatomie angepasste Konfiguration des Absorptionskörpers bei.

Die Cuffsockellinie des jeweiligen Cuffelements kann außerhalb und/oder oberhalb des Absorptionskörpers verlaufen. Es kann sich insbesondere als vorteilhaft erweisen, dass die jeweilige Cuffsockellinie innerhalb eines in der Längsrichtung mittleren Bereichs des Schrittabschnitts außerhalb des Absorptionskörpers, also neben Längsrändern des Absorptionskörpers verläuft und in einem vorderen und/oder hinteren Bereich des Schrittabschnitts innerhalb eines Randes des Absorptionskörpers, also oberhalb des Absorptionskörpers verläuft.

Es ist denkbar, dass die Cuffsockellinie entlang ihrer Erstreckung in Längsrichtung durchgehend gerade verläuft oder gerade und gekrümmte Abschnitte umfasst. Es erweist sich insbesondere als vorteilhaft, wenn der Inkontinenzartikel diesbezüglich so ausgebildet ist, dass die jeweilige Cuffsockellinie hinten und/oder vorn einen in Querrichtung nach innen, vorzugsweise bogenförmig nach innen, also in Richtung auf den unbefestigten freien Längsrand zu verlaufenden Cuffsockellinienabschnitt aufweist, welcher den emporerhebbaren Bereich des Cuffelements in der Längsrichtung begrenzt.

Hierdurch wird bei dem jeweiligen Cuffelement eine Tasche gebildet, also das Cuffelement nach hinten bzw. nach vorn abgeschlossen, so dass das Wandern von Ausscheidungen in Richtung Rückenabschnitt bzw. Bauchabschnitt minimiert werden kann.

Weiter trägt ein in Querrichtung, insbesondere bogenförmig verlaufender Cuffsockellinienabschnitt vorteilhaft zu einem planen Festlegen der Cuffelemente bei, was den Komfort für den Anwender unterstützt. Weiter kann mit einem solchen in Querrichtung, insbesondere bogenförmig verlaufenden Cuffsockellinienabschnitt den beim Anlegevorgang und Positionieren des Inkontinenzartikels am Anwender vorgenommenen Bewegungen in Querrichtung- bzw. Hüftumfangsrichtung und den damit einhergehenden Zug- und Reibungskräften auf das Cuffelement und den emporerhebbaren Bereich gut begegnet werden, da eine in Querrichtung verlaufende Fixierung des emporerhebbaren Bereichs des Cuffelements solchen Kräften weniger ausgesetzt ist.

Insbesondere bevorzugt ist vorgesehen, dass die jeweilige Cuffsockellinie im Bereich des nicht elastifizierten freien Längsrandendabschnittes einen in Querrichtung, insbesondere bogenförmig nach innen verlaufenden Cuffsockellinienabschnitt aufweist. Hierdurch wird die Taschenbildung im vorderen Bereich unterstützt und das männliche Genital noch besser aufgenommen und eingeschlossen.

Bei einer bevorzugten Ausführungsform, bei der die jeweilige Cuffsockellinie hinten und vorn einen in Querrichtung bogenförmig nach innen verlaufenden Cuffsockellinienabschnitt aufweist, kann es sich insbesondere als vorteilhaft erweisen, dass die Cuffsockellinie in dem Bereich zwischen den beiden in Querrichtung, insbesondere bogenförmig nach innen verlaufenden Cuffsockellinienabschnitten gerade verläuft.

Besonders vorteilhaft ist es dabei, wenn die jeweilige Cuffsockellinie über ihre im Wesentlichen gesamte Längserstreckung gerade erstreckt ist, und hiervon ausgehend hinten und/oder vorn einen zusätzlichen in Querrichtung, insbesondere bogenförmig nach innen, also in Richtung auf den unbefestigten freien Längsrand verlaufenden Cuffsockellinienabschnitt aufweist oder aufweisen kann, welcher den emporerhebbaren Bereich des Cuffelements in der Längsrichtung begrenzt. Eine solche Ausführung trägt vorteilhaft zu einer verstärkten Fixierung der Cuffelemente und damit zu deren Stabilität bei, da solchenfalls einem Kräfteeintrag durch Zug- und Reibungskräfte auf die Cuffelemente sowohl in Längs- als auch in Querrichtung begegnet werden kann.

Vorzugsweise weist der Schrittabschnitt außerhalb des Absorptionskörpers entlang der Beinöffnungen erstreckte Schrittelastifizierungsmittel auf, die als zusätzliche seitliche Auslaufsperre dienen und gleichzeitig das Anlegen des Schrittabschnitts gegen den Körper eines Trägers und eine bootförmige Krümmung des Schrittabschnitts fördern und hervorrufen. Hierdurch wird in Zusammenwirken mit den Elastifizierungsmitteln der Cuffelemente das Aufstellen der Cuffelemente und ihr Emporerheben in Richtung auf die Körperoberfläche befördert.

Vorzugsweise erstrecken sich die Schrittelastifizierungsmittel mit ihrem vorderen aktiven Ende weiter in Richtung des Bauchabschnitts als das vordere Ende des elastifizierten Abschnitts des freien Längsrandes der Cuffelemente. Solchenfalls können die Schrittelastifizierungsmittel auch in diesem Bereich das Aufstellen der Cuffelemente unterstützen.

Insbesondere bevorzugt ist vorgesehen, dass benachbart zum Längsende des Absorptionskörpers in Quer- oder Hüftumfangsrichtung elastisch dehnbare Bereiche im Bauchabschnitt ausgebildet sind, insbesondere dass der Schrittabschnitt einen in Richtung Bauchabschnitt und/oder Rückenabschnitt über ein Längsende des Absorptionskörpers in Längsrichtung hinauserstreckenden Überhang an Topsheet- und/oder Backsheet-Materiallagen aufweist, über welchen in Quer- oder Hüftumfangsrichtung elastisch dehnbare Bereiche im Bauchabschnitt ausgebildet sind. Hierdurch wird die Taschenbildung der Cuffelemente weiter gefördert, da eine bootartige Konfiguration des Inkontinenzartikels unterstützt wird.

Die für einen Inkontinenzartikel in Höschenform erforderliche elastische Dehnbarkeit in Quer- oder Hüftumfangsrichtung kann in vorteilhafter Weise dadurch realisiert werden, dass in dem Bauchabschnitt und/oder in dem Rückenabschnitt erste Elastifizierungsmittel vorgesehen sind, die sich in einem Abstand voneinander und im Wesentlichen in Quer- oder Hüftumfangsrichtung erstrecken und so den Bauchabschnitt und/oder den Rückenabschnitt flächenhaft elastifizieren, oder dass der Bauchabschnitt und/oder der Rückenabschnitt in Quer- oder Hüftumfangsrichtung elastisch dehnbare Flächenmaterialien umfasst. Insbesondere kann vorgesehen sein, dass der Bauchabschnitt und/oder der Rückenabschnitt zumindest außerhalb des Absorptionskörpers über die Längsrichtung im Wesentlichen durchgehend flächenhaft querelastifiziert sind. Eine Querelastifizierung ist also vom Grundsatz her dadurch möglich, dass im Wesentlichen fadenförmige Elastifizierungsmittel eingesetzt werden oder dass streifenförmige Elastifizierungsmittel oder alternativ auch zumindest in Querrichtung elastisch dehnbare Flächenmaterialien eingesetzt werden. In jedem Fall erweist es sich als vorteilhaft, wenn elastisch dehnbare Materialien mit im Wesentlichen undehnbaren Materialien, wie insbesondere Vliesmaterialien, kombiniert werden.

Es erweist sich insbesondere als vorteilhaft, wenn der Anteil der Fläche des Schrittabschnitts an der gesamten Fläche des Inkontinenzartikels 25 - 55 % beträgt, und dass der Schrittabschnitt wenigstens 12 %, insbesondere 12% - 45%, weiter insbesondere 15% -35%, weiter insbesondere 15% - 30% der Fläche des Bauchabschnitts und wenigstens 10 %, insbesondere 10% - 40%, weiter insbesondere 15% - 30%, weiter insbesondere 15% - 25% der Fläche des Rückenabschnitts überlappt.

Weiter vorteilhaft ist es, wenn die Überlappung des Schrittabschnitts mit dem Rückenabschnitt insbesondere kleiner ist als mit dem Bauchabschnitt. Eine im Bauchabschnitt verhältnismäßig größere Überlappung trägt positiv zur Verwendung als Männer-Inkontinenzartikel bei.

Die Gesamtlängserstreckung des Inkontinenzartikels beträgt vorzugsweise 700 - 1000 mm, weiter vorzugsweise 720 - 950 mm, weiter vorzugsweise 720 - 900 mm.

Die Querstreckung des Inkontinenzartikels beträgt vorzugsweise 500 - 1000 mm.

Insbesondere beträgt eine Fläche des Schrittabschnitts im eben ausgebreiteten Zustand 120.000 - 180.000 mm², weiter insbesondere 130.000 - 170.000 mm² und/oder eine Fläche des Absorptionskörpers im eben ausgebreiteten Zustand 50.000 - 100.000 mm², insbesondere 60.000 - 90.000 mm².

Der Schrittabschnitt weist vorzugsweise eine Längserstreckung von 500 - 800 mm, insbesondere von 500 - 700 mm auf. Eine maximale Erstreckung des Schrittabschnitts in Querrichtung beträgt vorzugsweise 160 - 300 mm, insbesondere 200 - 300 mm, weiter insbesondere 220 - 270 mm.

Der Schrittabschnitt weist vorzugsweise gerade Längsseitenkanten auf.

Der Absorptionskörper weist vorzugsweise eine Längserstreckung von 350 - 700 mm, weiter insbesondere von 400 - 600 mm auf und/oder eine Quererstreckung, also Breite, vorzugsweise eine maximale Quererstreckung von 150 - 210 mm, insbesondere von 160 - 200 mm auf. Insbesondere vorteilhaft ist eine konturierte Ausbildung des Absorptionskörpers mit einem vorzugsweise zwischen dessen Endbereichen vorhandenen mittleren Bereich von geringerer Quererstreckung. Weiter insbesondere ist der vordere Endbereich in seiner Querstreckung größer als der hintere Endbereich des Absorptionskörpers.

Insbesondere bevorzugt weist der Absorptionskörper einen Flächenanteil an der vorderen Produkthälfte von mindestens 15%, insbesondere 18 - 35%, weiter insbesondere 20 - 30% auf.

Insbesondere vorteilhaft ist es, wenn der Absorptionskörper 5-25%, insbesondere 5-20%, weiter insbesondere 5-15% der Fläche des Bauchabschnitts und/oder 4 - 20%, insbesondere 4-15%, weiter insbesondere 4-10% der Fläche des Rückenabschnitts überlappt.

An dieser Stelle sei ausgeführt, dass in der vorliegenden Anmeldung jegliche Bemessungsangaben hinsichtlich Quererstreckung, Längserstreckung oder Abstand von Abschnitten oder an von an sich beliebigen Komponenten des Artikels im eben ausgebreiteten Zustand der den Artikel bildenden Flachmaterialien bestimmt werden, also gegebenenfalls nach Auftrennung der herstellerseitig schon angebrachten Seitennähte, so dass der betreffende Artikel in die in den Figuren dargestellte ebene Konfiguration gebracht werden kann.

Wenn der Artikel beispielsweise durch fadenförmige Elastifizierungsmittel im sogenannten "Stretchbondverfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies/Folien-Verbundbasis auf natürliche Weise wie in den Figuren dargestellt.

Bei dem hier in Rede stehenden dreiteiligen Aufbau des Inkontinenzartikels erweist es sich insbesondere als vorteilhaft, wenn der Schrittabschnitt gewissermaßen als unitäre Einheit, vorzugsweise mit eingebrachten und fixierten Cuffelementen und vorzugsweise einschließlich der Schrittelastifizierungsmittel ausgebildet und als solche bezüglich des Bauchabschnitts und des Rückenabschnitts in Überlappung positioniert und fixiert wird. Hierfür erweist es sich als vorteilhaft, dass der Schrittabschnitt ein flüssigkeitsundurchlässiges Backsheet-Material und ein flüssigkeitsdurchlässiges Topsheet-Material umfasst, zwischen denen der Absorptionskörper angeordnet ist. Hierbei können das Backsheet-Material und/oder das Topsheet-Material in Querrichtung einen Überhang über den Absorptionskörper bilden. Solchenfalls erweist es sich als vorteilhaft, wenn die Schrittelastifizierungsmittel zumindest mit dem Topsheet-Material oder mit dem Backsheet-Material oder mit einem in Querrichtung nach außen verlaufenden Bereich des Materialabschnitts, welcher das Cuffelement bildet, verbunden sind.

Die Cuffelemente umfassen vorzugsweise ein Vliesmaterial, insbesondere ein hydrophobes Vliesmaterial, oder sind daraus gebildet.

Das Flächengewicht des hierfür eingesetzten Vliesmaterials beträgt vorzugsweise 6-20 g/m², insbesondere 6-15 g/m², weiter insbesondere 8-15 g/m², weiter insbesondere 10-15 g/m².

Der Inkontinenzartikel in Höschenform ist insbesondere ein Erwachsenen-Inkontinenzartikel. Insbesondere ist der Inkontinenzartikel für die Verwendung durch Männer vorgesehen, ist somit insbesondere ein Männer-Inkontinenzartikel.

Gegenstand der Erfindung ist auch eine Anordnung (Array) aus einem ersten und einem zweiten Inkontinenzartikel, die einander im Hinblick auf verschiedene Benutzungssituationen in vorteilhafter Weise ergänzen und die sich in einer oder in mehrfacher Hinsicht voneinander unterscheiden.

Die Anordnung (Array) ergibt sich dabei durch die sinnfällige Herrichtung der zum Array gehörenden Inkontinenzartikel. Insbesondere durch die Beziehung oder das Verhältnis der Artikel zueinander. Diese erfolgt entweder durch Darbietung in einer gemeinsamen Verpackungseinheit und/oder bevorzugt durch die Anbringung von Kennzeichnungen auf den Inkontinenzartikeln und/oder deren Verpackung und/oder die Darbietung in räumlicher oder inhaltlicher Nähe zueinander, die die Zugehörigkeit zu einem Array kenntlich machen. Die den Array bildenden Artikel stammen bevorzugt von ein und demselben Hersteller.

Die einen Array bildenden Inkontinenzartikel weisen bevorzugt dieselbe Produktkennzeichnung auf, wie eben Markennamen und/oder Sub-Markennamen.

Eine Anordnung aus einem ersten Inkontinenzartikel und einem zweiten Inkontinenzartikel wird verstanden als zumindest jeweils einem Vertreter davon und schließt eine Mehrzahl des einen ersten und/oder des einen zweiten Inkontinenzartikels ein.

Erfindungsgemäß wird vorliegend daher eine Anordnung (Array) aus einem ersten Inkontinenzartikel insbesondere mit den Merkmalen nach einem oder mehreren der Ansprüche 1 bis 18 sowie der vorstehenden Beschreibung und der Figurenbeschreibung und einem zweiten Inkontinenzartikel mit den im Anspruch 19 genannten Merkmalen beansprucht. Die beiden Artikel unterscheiden sich also hinsichtlich der elastischen Ausbildung des emporerhebbaren Bereichs der Cuffelemente und insbesondere auch der Anordnung des Absorptionskörpers. Dabei bildet der Artikel mit einer relativ zur Quermittelachse des Inkontinenzartikels nach vorn verschobenem Absorptionskörper und in Längsrichtung größeren nicht elastifizierten Längsrandendabschnitten der Cuffelemente den ersten Inkontinenzartikel und der Artikel mit demgegenüber weitergehend elastifzierten Cuffelementen und weniger nach vorne verschobenem, also weiter hinter angeordnetem Absorptionskörper den zweiten Inkontinenzartikel. Es hat sich gezeigt, dass gerade bei einer Verwendung des betreffenden ersten Inkontinenzartikels durch Männer eine teilweise Nichtelastifizierung der freien Längsränder der Cuffelemente im vorderen Bereich, durch die sogenannten nicht elastifizierten freien Längsrandendabschnitte, bei nach vorne verschobenen Absorptionskörpern als angenehmer empfunden wird, während demgegenüber eine weitgehend durchgehend längselastische Ausbildung des Längsrandes des emporerhebbaren Bereichs der Cuffelemente und ein weiter mittig angeordneter Absorptionskörper als geeigneter für weibliche Benutzer empfunden wird und funktional wirksam sein kann.

Die Längserstreckung des nicht elastifizierten freien Längsrandabschnitt C1 in Bezug auf eine Längserstreckung C2 des unbefestigten freien Längsrands der Cuffelemente in der sich von der Quermittelachse zum Bauchabschnitt erstreckenden Produkthälfte ist im ersten Inkontinenzartikel größer als im jeweils zweiten Inkontinenzartikel. Solchenfalls ist auch eine Ausführungsform des zweiten Inkontinenzartikels ohne einen vorderen nicht elastifizierten freien Längsrandabschnitt eingeschlossen. Der Unterschied zeigt sich insbesondere derart, dass das Verhältnis C1/C2 im ersten Inkontinenzartikel insbesondere um einen Faktor von mindestens 2, insbesondere mindestens 3, insbesondere mindestens 4, weiter insbesondere mindestens 5, weiter insbesondere um den Faktor von höchstens 10, weiter insbesondere höchstens 9 größer ist.

Insbesondere bevorzugt werden innerhalb eines Arrays ein erster und zweiter Inkontinenzartikel von im Wesentlichen gleicher Produktlänge herangezogen.

Als eine im Wesentlichen gleiche Produktlänge wird hierin verstanden, dass eine Abweichung der Produktlängserstreckung des ersten und zweiten Inkontinenzartikels um höchstens 10%, insbesondere höchstens 8%, weiter insbesondere höchstens 6% voneinander zulässig ist.

Insbesondere kann weiter vorgesehen sein, dass im ersten Inkontinenzartikel die Längserstreckung des nicht elastifizierten freien Längsrandabschnitt C1 der Cuffelemente in Bezug auf eine Gesamtlängserstreckung C3 des unbefestigten freien Längsrands der Cuffelemente, also C1/C3, größer ist als im jeweils zweiten Inkontinenzartikel.

Der Unterschied zeigt sich insbesondere derart, dass das Verhältnis C1/C3 im ersten Inkontinenzartikel insbesondere um einen Faktor von mindestens 2, insbesondere von mindestens 3, insbesondere mindestens 4, weiter insbesondere mindestens 5, weiter insbesondere höchstens um den Faktor 12, insbesondere höchstens 10 größer ist.

Der vorangehend genannte Unterschied ist insbesondere bei einem ersten und zweiten Inkontinenzartikel bei im Wesentlicher gleicher Produktlänge der beiden Inkontinenzartikel gegeben, wobei die Produktlängen des ersten und zweiten Inkontinenzartikels um maximal 10%, insbesondere maximal 8%, weiter insbesondere maximal 6% voneinander abweichen.

Weiterhin betrifft die Erfindung eine Anordnung (Array) aus einem ersten Inkontinenzartikel nach einem oder mehreren der vorstehenden Ansprüche und aus mindestens einem zweiten Inkontinenzartikel, wobei die Cuffelemente der ersten Inkontinenzprodukte im Bereich der Quermittelachse des Inkontinenzartikels eine größere Höhe aufweisen als die des zweiten Inkontinenzartikels.

Die nachfolgenden Merkmalsaufzählungen können sich in beliebiger Kombination mit vorstehend erläuterten Merkmalen eines Inkontinenzartikels, insbesondere sowohl für einen ersten als auch einen zweiten Inkontinenzartikel als vorteilhaft erweisen:
- dass der Bauchabschnitt und/oder der Rückenabschnitt vorzugsweise eine von der Quer- oder Hüftumfangsrichtung abweichende und in Richtung auf eine Quermittelachse des Schrittabschnitts zulaufende Randkontur zur Begrenzung der Beinöffnungen aufweisen.
- dass in einem schrittseitigen und den Beinöffnungen zugewandten Bereich des Bauchabschnitts und des Rückenabschnitts vorzugsweise zweite Elastifizierungsmittel vorgesehen sind, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.

- dass ein minimaler Abstand der zweiten Elastifizierungsmittel voneinander in den Seitennahtbereichen vorzugsweise 3 - 8 mm, insbesondere 3 - 7 mm und weiter insbesondere 3 - 6 mm beträgt.
- dass ein maximaler Abstand der zweiten Elastifizierungsmittel voneinander an einem Absorptionskörperrand oder an einem Längsrand des Schrittabschnitts vorzugsweise 10 - 35 mm, insbesondere 12 - 35 mm und weiter insbesondere 15 - 32 mm beträgt.
- dass der maximale Abstand der zweiten Elastifizierungsmittel voneinander an einem Absorptionskörperrand oder an einem Längsrand des Schrittabschnitts im Rückenabschnitt vorzugsweise größer ist als im Bauchabschnitt.
- dass die ersten und/oder zweiten Elastifizierungsmittel vorzugsweise in einem Überlappungsbereich mit dem Absorptionskörper des Schrittabschnitts hinsichtlich ihrer elastischen Eigenschaften deaktiviert sind.
- dass der Abstand des schrittzugewandten innersten ersten oder zweiten Elastifizierungsmittels des Bauchabschnitts von dem entsprechenden schrittzugewandten innersten ersten oder zweiten Elastifizierungsmittel des Rückenabschnitts vorzugsweise 220 - 420 mm, insbesondere 220 - 400 mm, weiter insbesondere 220 - 350 mm beträgt.
- dass der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts vorzugsweise 2 - 40 mm, vorzugsweise 3 - 30 mm, insbesondere vorzugsweise 4 - 15 mm beträgt.
- dass die Erstreckung des Bauchabschnitts und des Rückenabschnitts im Seitennahtbereich in Längsrichtung vorzugsweise wenigstens 100 mm, insbesondere wenigstens 120 mm und insbesondere 120 mm - 220 mm beträgt.

- dass der minimale Abstand des Bauchabschnitts und des Rückenabschnitts in Längsrichtung voneinander vorzugsweise 200 - 450 mm, insbesondere 200 - 420 mm beträgt.
- dass der Schrittabschnitt vorzugsweise ein flüssigkeitsundurchlässiges Backsheet-Material und ein Topsheet-Material umfasst, zwischen denen der Absorptionskörper angeordnet ist, wobei das Backsheet-Material und/oder das Topsheet-Material in Querrichtung einen Überhang über den Absorptionskörper bilden können und dieser Überhang
- in der Summe beidseits des Absorptionskörpers, also in der Summe links und rechts - vorzugsweise wenigstens 18 %, insbesondere 20 - 35 % und weiter insbesondere 25 - 32 % bezogen auf die größte Breite des Schrittabschnitts beträgt.
- dass die Schrittelastifizierungsmittel in Längsrichtung vorzugsweise vor den ersten und/oder zweiten Elastifizierungsmitteln enden.
- dass die Schrittelastifizierungsmittel in Längsrichtung vorzugsweise den Bauchabschnitt und/oder den Rückenabschnitt überlappen.
- dass der Bauchabschnitt und der Rückenabschnitt zumindest außerhalb des Absorptionskörpers über die Längsrichtung vorzugsweise im Wesentlichen durchgehend flächenhaft querelastifiziert sind.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels.

In der Zeichnung zeigt:
- Figur 1: einen Inkontinenzartikel in ausgebreiteter Konfiguration gemäß der Erfindung,
- Figur 2: einen Schnitt durch einen Inkontinenzartikel gemäß Figur 1
- Figur 3a und b: Schnittdarstellungen des Inkontinenzartikels,
- Figur 4 a-d: Cuffelemente an verschiedenen Stellen des Inkontinenzartikels,
- Figur 5: Cuffelemente an verschiedenen Stellen des Inkontinenzartikels,
- Figur 6: einen Array gemäß der Erfindung.
- Figur 7: eine Draufsicht auf einen Inkontinenzartikel mit eingezeichneten Abmessungen

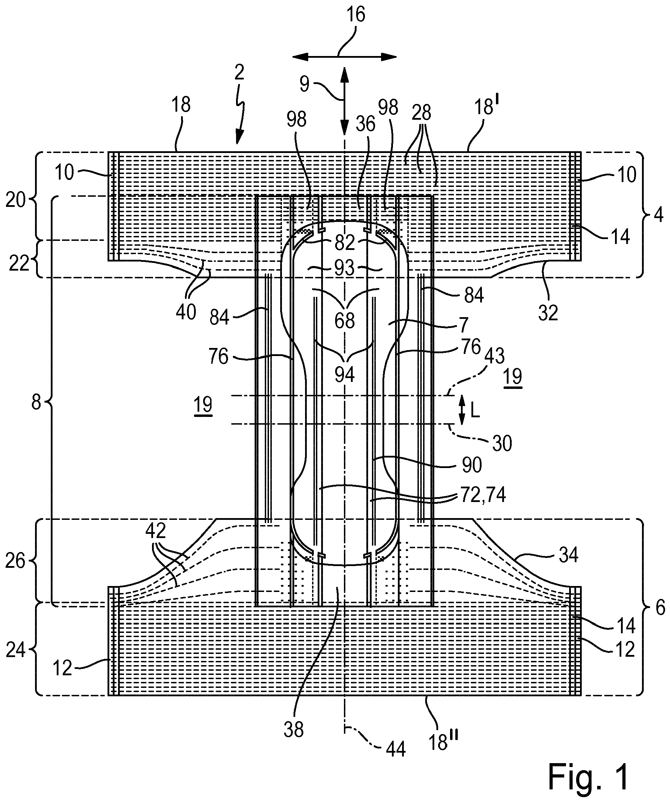

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem Flächenanteil des Bauchabschnitts 4 einerseits und des Rückenabschnitts 6 andererseits überlappt und im Überlappungsbereich 36, 38 herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 mit einer vorderen 18' und einer hinteren 18'' Hüftöffnungskante und Beinöffnungen 19, durch welche hindurch der Benutzer den Inkontinenzartikel wie ein Höschen anlegt.

Der Bauchabschnitt 4 lässt sich bei der beispielhaften Ausführungsform in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra^{®}-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Inkontinenzartikels zulaufende Randkontur 32 bzw. 34. Diese Randkontur 32, 34 ist abschnittsweise bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus einer bevorzugten Ausführungsform in den Figuren zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt.

Darüber hinaus ist vorgesehen, dass der Absorptionskörper 7 in Richtung auf die Hüftöffnungskante 18' des Bauchabschnitts 4 nach vorne verschoben ist, so dass seine Quermittellinie 43, die sich ebenfalls aus der Halbierung der Länge des Absorptionskörpers 7 ergibt, gegenüber der Quermittelachse 30 in Richtung auf den Bauchbereich um eine Länge L verschoben ist. Durch diese Vorverlagerung des Absorptionskörpers 7 in Richtung auf den Bauchabschnitt 4 wird der männlichen Anatomie und dem Ort des Flüssigkeitsanfalls bei einem männlichen Benutzer verstärkt Rechnung getragen.

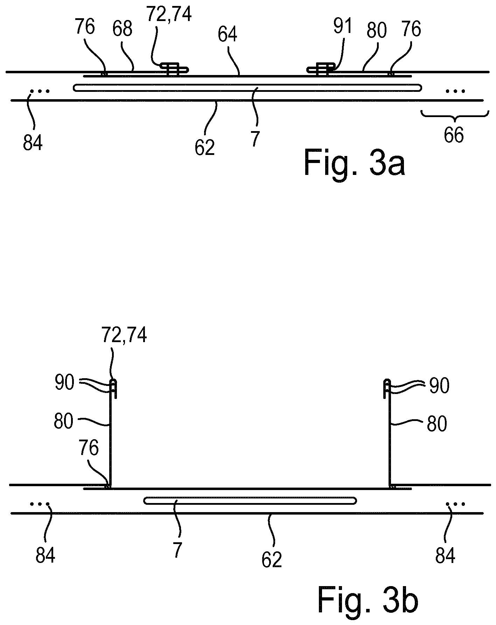

Der Schrittabschnitt 8 umfasst ein flüssigkeitsundurchlässiges Backsheet 62, das insbesondere von einem atmungsaktiven, jedoch flüssigkeitsdichten Folienmaterial gebildet sein kann, und ein, vorzugsweise auf Vliesbasis beruhendes flüssigkeitsdurchlässiges Topsheet 64. Zwischen dem Backsheet 62 und dem Topsheet 64 ist wie aus den Figuren 3 ersichtlich der Absorptionskörper 7 angeordnet. Im beispielhaft dargestellten Fall bildet das Backsheet 62 in Querrichtung 16 einen Überhang 66 über den Absorptionskörper 7. Das Topsheet 64 überragt den Absorptionskörper 7 in Querrichtung nur verhältnismäßig geringfügig; jedoch ist beidseits und entlang des Absorptionskörpers 7 in Längsrichtung 9 verlaufend jeweils ein aufstehendes Barrieremittel vorgesehen, welches als aufstehendes Cuffelement 68 bezeichnet wird und vorzugsweise aus einem hydrophoben, insbesondere flüssigkeitsundurchlässigen Vliesstoffmaterial gebildet ist.

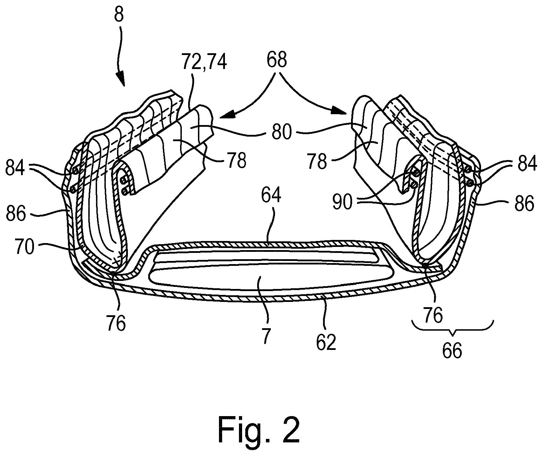

Das Cuffelement 68 ist aus einem auf sich selbst entlang der Längsrichtung 9 umgefalteten Vliesmaterialabschnitt 70 gebildet, wobei eine hierbei entstehende Faltlinie 72 einen unbefestigten freien Längsrand 74 bildet, der aber wie in Figur 2 gezeigt verrundet ausgebildet ist. Es wird daher eine Art Tunnel ausgebildet.

Der auf sich selbst umgefaltete Vliesmaterialabschnitt 70 ist auf die körperzugewandte Seite von Topsheet und/oder Backsheet aufgebracht und dort entlang einer sogenannten Cuffsockellinie 76 mit dem Topsheet 64 oder Backsheet 62 gefügt. Weiter wird hierdurch in Querrichtung innerhalb dieser Cuffsockellinie 76 ein emporerhebbarer Bereich 80 des Cuffelements 68 begrenzt und definiert. Dieser emporerhebbare Bereich 80 des Cuffelements 68 umfasst über einen bestimmten Bereich ein Elastifizierungsmittel 90, beispielsweise zwei fadenförmige Elastifizierungsmittel, wie Lycra^{®}-Fäden, die in dem umgefalteten Bereich 78 im vorgedehnten Zustand, im sogenannten Stretch-Bond-Verfahren, mit dem Material der Cuffelemente 68 verbunden worden sind. Die Elastifizierungsmittel 90 dienen dazu, das Aufstellen und Emporerheben der Cuffelemente 68 insbesondere bei breiten Absorptionskörpern 7, wie sie bei bevorzugt für Männer verwendbaren Produkten eingesetzt werden, zu verbessern. Die in Figur 2 gezeigte Gestaltung stellt dabei eine Gestaltung im Bereich der Quermittelachse 30 dar.

Im beispielhaft bevorzugt dargestellten Fall, wie in Figur 1 dargestellt, erstreckt sich die Cuffsockellinie 76 im Wesentlichen über die gesamte Längserstreckung des Cuffelements 68 in einer geraden Linie. Es ist jedoch hinten und vorn ausgehend von der geraden Erstreckung ein zusätzlicher in Querrichtung 16 bogenförmig nach innen, also in Richtung auf den unbefestigten freien Längsrand 74 verlaufender Cuffsockellinienabschnitt 82 ausgebildet, der den emporerhebbaren Bereich 80 des Cuffelements 68 nach hinten und nach vorn begrenzt. Es wird auf diese Weise eine Art Tasche gebildet.

In Querrichtung außerhalb und beidseits des Absorptionskörpers 7 sind in der Längsrichtung 9 verlaufende und der jeweiligen Beinöffnung 19 zugeordnete Schrittelastifizierungsmittel 84 vorgesehen. Sie sind im beispielhaft dargestellten Fall mit einem Querabstand zur Cuffsockellinie zwischen dem Vliesmaterialabschnitt 70 des Cuffelements 68 und dem Backsheet-Material 62 angeordnet. Sie vermögen somit den Schrittabschnitt 8 in der Längsrichtung 9 zu elastifizieren und eine Raffung der Chassismaterialien des Schrittabschnitts 8 herbeizuführen. In der Gebrauchssituation, die in Figur 2 schematisch dargestellt ist, erheben sich daher die Längsrandbereiche 86 des Schrittabschnitts 8 gegen die Beine des Benutzers und bilden einen Seitenauslaufschutz des Inkontinenzartikels. Durch die hiermit verbundene Bootform des Schrittabschnitts 8 wird das Aufrichten der emporerhebbaren Bereiche 80 der jeweiligen Cuffelemente 68, was in Figur 2 schematisch dargestellt ist, weiter unterstützt.

Die Figuren 3a und b zeigen Schnittansichten (jeweils schematisch) des Schrittabschnitts 8 im eben ausgebreiteten Zustand. Dabei zeigt Figur 3a einen Schnitt durch den Schrittbereich 8 in dem Bereich, in dem die Cuffsockellinie 76 bogenförmig nach innen verläuft, so dass die emporerhebbaren Bereiche 80 auf dem Topsheetmaterial 64 z-förmig gefaltet auf sich selber durch das Element 91 fixiert werden. Figur 3b zeigt dahingegen einen Schnitt, in dem die emporerhebbaren Bereiche 80 aufgerichtet sind, die Schnittebene liegt hier im Bereich der Quermittelachse 30 und zeigt die maximale Aufrichtung bei gleichzeitig elastifiziertem Längsrandbereich 74 der Cuffelemente 68. Um den anatomischen Gegebenheiten eines männlichen Trägers und dem dazu erfindungsgemäß erkannten Komfort Rechnung zu tragen,
ist der freie Längsrand 74 im vorderen Bereich der Cuffelemente mit einem freien nicht elastifizierten Längsrandendabschnitt 93 versehen, der zwischen dem elastischen Abschnitt 94 des Längsrandes und dem fixierten Endrandabschnitt 98 angeordnet ist, wie in Figur 1 dargestellt.

Der nichtelastifizierte Längsrandendabschnitt weist hierbei eine Längserstreckung von wenigstens 8% der Längserstreckung des unbefestigten freien Längsrands der Cuffelemente 68 auf. Eine Längserstreckung des nicht elastifizierten Längsrandendabschnitts 93 von wenigstens 12% bis höchstens 45% der Längserstreckung des unbefestigten freien Längsrand der Cuffelemente in der vorderen Produkthälfte wurde als vorteilhaft erkannt, um sowohl Komfort als auch Funktionsfähigkeit der Cuffelemente in einem Männerprodukt bereitzustellen.

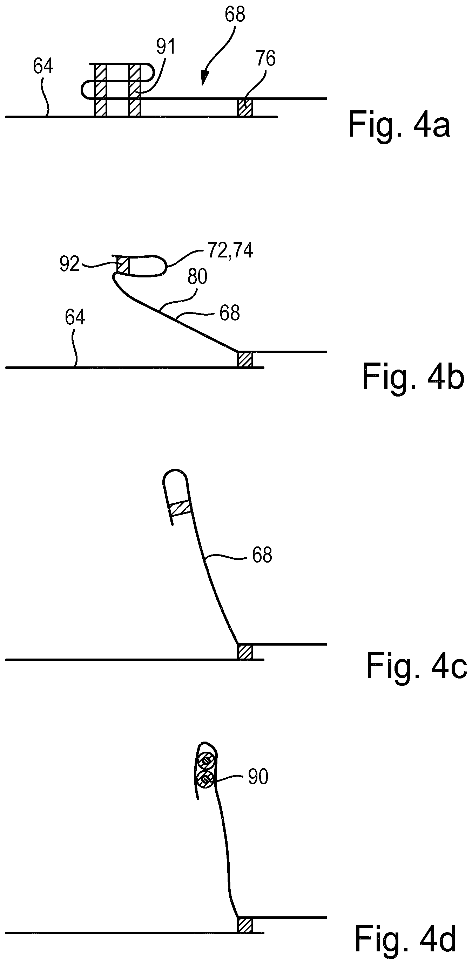

Dieser Anteil stellt sich insbesondere vorteilhaft bei einer bevorzugten Ausführungsform mit einer C/Z-förmigen Rückfaltung des freien Längsrandes im fixierten Endrandabschnitt dar. Solchenfalls kann das Cuffelement im Anwendungszustand innerhalb dieser Erstreckung bis zum Übergang zum elastifizierten Längsrand mit mittels der Elastifizierungsmittel 90 aufgerichteten Zustandes der Cuffelemente (Figur 4d) eine plane und damit weiche und so für den Anwender eine komfortunterstützende Auflagefläche des Cuffelements (Figur 4b) bieten, wie in der verschiedenen Gestaltung der Cuffelemente 68 in Figur 4a bis d exemplarisch dargestellt ist. Dabei zeigt Figur 4a eine schematische Darstellung der Cuffelemente mit der Cuffsockelfixierung 76 sowie der Fixierung des z-förmig gefalteten fixierten Längsrandbereiches mit Fixierelementen 91 auf dem Topsheet 64, wie es beispielhaft auch in Figur 3a dargestellt ist. Durch diese z-förmige Faltung wird das Aufstellen der Taschen in einem mittleren Bereich der Cuffelemente 68 erleichtert. In einem hieran anschließenden Bereich, in dem die Cuffelemente 68 nicht mehr auf dem Topsheet 64 fixiert sind, ist im in Richtung auf den Bauchbereich 4 gerichteten vorderen Abschnitt der Cuffelemente 68 eine Gestaltung vorgesehen, wobei die Cuffelemente im Bereich der Faltlinie 72 sowie des unbefestigten freien Längsrands 74 einen nichtelastifizierten Längsrandendabschnitt 93 aufweisen. Durch die Nichtelastifizierung in diesem nach vorne gerichteten Bereich der Cuffelemente 68 erfolgt in diesem Längsrandendabschnitt 93 keine Raffung oder Rüschung der Cuffelemente 68, so dass nicht die Gefahr von Hautirritationen und eine negative Zusammenwirkung mit einer Behaarung eines Benutzers zu befürchten ist (Figur 4b). Figur 4c zeigt nun eine Ausgestaltung, bei der die Cuffelemente bereits weiter aufgerichtet sind, jedoch noch der unelastifizierte Abschnitt 93 der Cuffelemente 68 dargestellt ist. In diesem Bereich sind die Cuffelemente 68 lediglich bezüglich ihres Faltbereichs, hier sehr vereinfacht dargestellt, über eine Fixierung 92 umgeschlagen, so dass sich die Faltlinie 72 ausbildet.

Figur 4d zeigt nun einen Schnitt in dem Bereich, in dem eine Elastifizierung über die Elastifizierungsmittel 90 der Cuffelemente erfolgt.

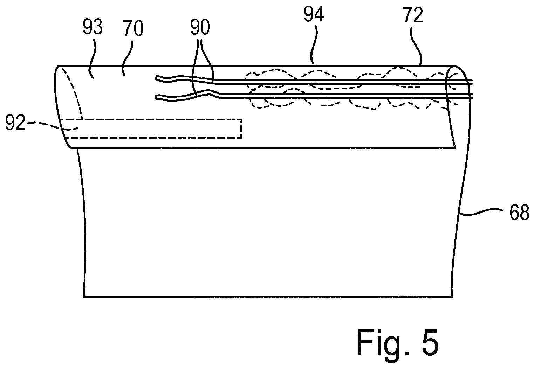

Figur 5 zeigt dabei nochmal den Bereich eines Cuffelements 68 mit der Faltlinie 72 sowie dem umgefalteten Bereich 70 im Übergang vom elastifizierten Abschnitt 94 in den Längsrandendabschnitt 93, der nicht elastifiziert ausgebildet ist. Die im Abschnitt 94 eingebrachten und dort aktiven Elastifizierungsmittel 90 (so beispielsweise mittels einer Fadenbeleimung innerhalb des umgefalteten Bereichs 70 auf das Cuffelement festgelegt) können im Längsrandendabschnitt 93 zwar noch vorhanden sein, sind aber ihrer elastischen Aktivität genommen. In diesem Bereich 93 wird dann der umgefaltete Materialabschnitt 70 über ein Fixierungsmittel 92 vorzugsweise nur randständig, ohne weitere Fügestellen auf sich selber fixiert.

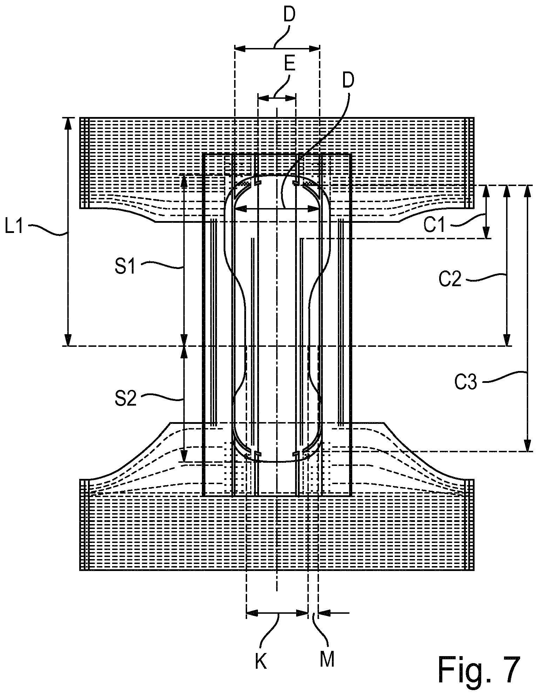

Die Figur 7 zeigt einen Inkontinenzartikel im eben ausgebreiteten Zustand und verdeutlicht folgende Bemessungen:
- Längserstreckung C1 des unelastifizierten Längsrandendabschnitts;
- Gesamtlängserstreckung C3 des unbefestigten freien Längsrandes der Cuffelemente und dessen Längserstreckung C2 ab der Quermittelachse des Inkontinenzartikels;
- Länge Absorptionskörper S1 ab der Quermittelachse in Richtung Bauchabschnitt und S2 in Richtung Rückenabschnitt;
- Länge L1 des Inkontinenzartikels ausgehend von der Quermittelachse bis zur Vorderkante des Bauchabschnitts;
- Abstand E zwischen freien Kanten der Cuffelemente am fixierten Endrandabschnitt;
- maximaler Querabstand D der Cuffsockellinien 76 voneinander, dabei nächstgelegen zum fixierten Endrandabschnitt;
- Abstand M zwischen Cuffsockellinie und Längsrand Absorptionskörper an dessen minimalen Breite;
- Breite K des Absorptionskörpers 7; hierbei minimale Breite.

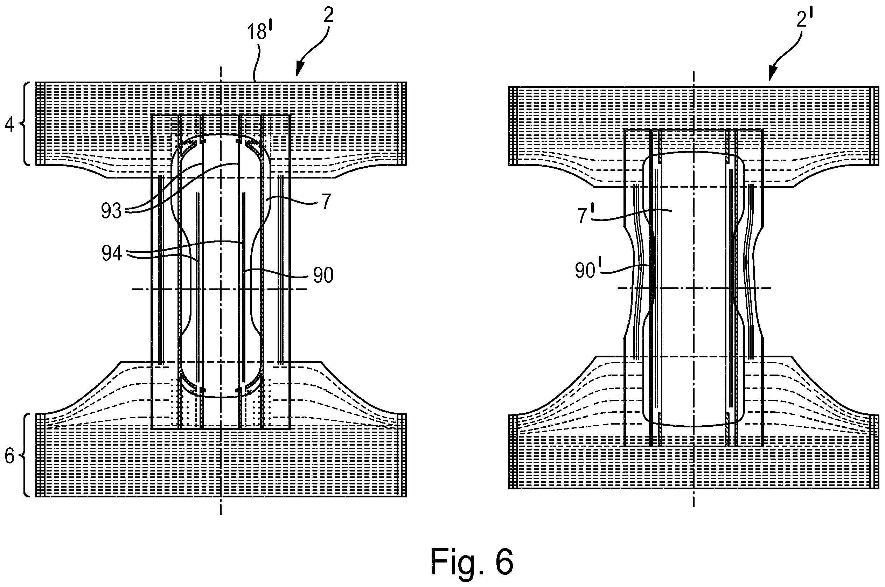

Schließlich zeigt Figur 6 eine Draufsicht auf einen weiteren zweiten Inkontinenzartikel 2', der sich von dem bislang beschriebenen Inkontinenzartikel 2, der ebenfalls dargestellt ist, insbesondere dadurch unterscheidet, dass die Quermittellinie seines Absorptionskörper 7' weniger weit in Richtung auf die Hüftöffnungsvorderkante 18' des Bauchabschnitts 4 nach vorne verschoben ist als beim Artikel 2. Darüber hinaus ist der zweite Inkontinenzartikel 2' so ausgebildet, dass die Elastifizierungsmittel 90' sich über im Wesentlichen die gesamte Längserstreckung der freien Längsrandbereiche der Cuffelemente 68 erstrecken und insbesondere kein entsprechend dem Inkontinenzartikel 2 ausgebildeter Abschnitt 93 vorgesehen ist, der unelastifiziert ist. Durch diese beiden Maßnahmen wird ein Inkontinenzartikel 2 geschaffen, der insbesondere an die Bedürfnisse und anatomischen Gegebenheiten eines männlichen Trägers angepasst ist, im Vergleich zu einem Inkontinenzartikel 2', der als zweiter Inkontinenzartikel vorzugsweise an die Bedürfnisse eines weiblichen Trägers angepasst ist. Die beiden Inkontinenzartikel zusammen bilden eine beanspruchte Anordnung ("Array"). Darüber hinaus unterscheiden sich die Inkontinenzartikel 2 und 2' durch die Formgebung des absorbierenden Körpers 7, der beim ersten Inkontinenzartikel 2 im zum Bauchabschnitt 4 gerichteten Bereich eine größere Breite in Querrichtung 16 aufweist als im hinteren Bereich, wohingegen der Absorptionskörper 7' des zweiten Inkontinenzartikels 2' im vorderen und hinteren Bereich jeweils gleich breit ausgebildet ist und dadurch an den eher mittigen Flüssigkeitsanfall eines weiblichen Benutzers angepasst ist. Weiterhin ist der Überlappungsbereich des Schrittabschnitts 8 mit dem Bauchabschnitt 4 beim ersten Inkontinenzprodukt 2 größer als beim zweiten Inkontinenzprodukt, wodurch auch hier der weiter vorne auftretende Flüssigkeitsanfall bei einem männlichen Benutzer besser abgebildet wird.

## Patentansprüche

1. Inkontinenzartikel (2) in Höschenform mit einer Längsmittelachse (44) und einer Quermittelachse (30) für die Aufnahme von Körperausscheidungen für die bevorzugte Verwendung durch Männer, mit einem in einer Längsrichtung (9) voneinander beabstandeten vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung (16) geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in einer Längsrichtung (9) zwischen Bauchabschnitt (4) und Rückenabschnitt (6) erstreckt und an den Bauchabschnitt (4) und den Rückenabschnitt (6) in einem jeweiligen Überlappungsbereich unlösbar angefügt ist, wobei der Schrittabschnitt (8), der Bauchabschnitt (4) und der Rückenabschnitt (6) gemeinsam Beinöffnungen (19) des Inkontinenzartikels (2) begrenzen und wobei der Schrittabschnitt (8) beidseits eine seitliche Auslaufsperre bildende und beidseits entlang der Längserstreckung des Absorptionskörpers (7) verlaufende Cuffelemente (68) aufweist, die zumindest entlang einer Cuffsockellinie (76) an der körperzugewandten Seite des Artikels (2) festgelegt sind und einen unbefestigten freien Längsrand (74) aufweisen, der zumindest abschnittsweise in der Längsrichtung elastifiziert ist, mit dem sich die Cuffelemente (68) von der körperzugewandten Seite des Inkontinenzartikels (2) emporerheben können und so die jeweilige seitliche Auslaufsperre bilden und wobei der Absorptionskörper (7) so angeordnet ist, dass eine Quermittellinie (43) des Absorptionskörpers (7) zwischen der Quermittelachse (30) des Inkontinenzartikels (2) und einer die Hüftöffnung (18) begrenzenden Hüftöffnungskante (18') des Bauchabschnitts (4) angeordnet ist, **dadurch gekennzeichnet, dass** der jeweilige unbefestigte freie Längsrand (74) der beiden Cuffelemente (68) zum vorderen Bauchabschnitt (4) hin einen nicht elastifizierten freien Längsrandendabschnitt (93) aufweist, dessen Längserstreckung C1 wenigstens 12%, insbesondere mindestens 15%, insbesondere mindestens 20% und insbesondere höchstens 45%, insbesondere höchstens 40%, weiter insbesondere höchstens 35% einer Längserstreckung C2 des unbefestigten freien Längsrands (74) der Cuffelemente (68) in der sich von der Quermittelachse (30) des Inkontinenzartikels (2) zum Bauchabschnitt (4) erstreckenden Produkthälfte beträgt.

2. Inkontinenzartikel nach Anspruch 1, **dadurch gekennzeichnet, dass** der jeweilige unbefestigte freie Längsrand (74) der beiden Cuffelemente (68) zum vorderen Bauchabschnitt (4) hin einen nicht elastifizierten freien Längsrandendabschnitt (93) aufweist, dessen Längserstreckung C1 wenigstens 8%, insbesondere wenigstens 10% und insbesondere wenigstens 12% sowie insbesondere höchstens 30% insbesondere höchstens 28%, insbesondere höchstens 25% und insbesondere höchstens 23% einer Gesamtlängserstreckung C3 des unbefestigten freien Längsrands (74) der Cuffelemente (68) beträgt.

3. Inkontinenzartikel nach Anspruch 1 oder 2, **dadurch gekennzeichnet, dass** die jeweilige Cuffsockellinie (76), insbesondere im Bereich des nicht elastifizierten freien Längsrandendabschnitts (93), einen in Querrichtung (16), insbesondere bogenförmig, nach innen in Richtung auf eine Längsmittelachse (44) des Inkontinenzartikels (2) und damit in Richtung auf den freien Längsrand (76) des jeweiligen Cuffelements (68) zu verlaufenden Cuffsockellinienabschnitt (76, 82) aufweist, wobei dieser Cuffsockellinienabschnitt (76, 82) den emporerhebbaren Bereich (74) des Cuffelements (68) in der Längsrichtung begrenzt.

4. Inkontinenzartikel nach einem der vorstehenden Ansprüche, **dadurch gekennzeichnet, dass** das Verhältnis einer Länge S1 des Absorptionskörpers (7) vor der Quermittelachse (30) des Inkontinenzartikels (2) in Richtung Bauchabschnitt (4) zu einer Länge S2 des Absorptionskörpers (7) hinter der Quermittelachse (30) des Inkontinenzartikels (2) in Richtung Rückenabschnitt (6) mindestens 1,2, vorzugsweise mindestens 1,3 und höchstens 2,0, insbesondere höchstens 1,8 und insbesondere höchstens 1,6 beträgt.

5. Inkontinenzartikel nach einem der vorstehenden Ansprüche, **dadurch gekennzeichnet, dass** das Verhältnis einer Länge S1 des Absorptionskörpers (7) vor der Quermittelachse (30) in Richtung Bauchabschnitt (4) zu einer Länge L1 des Inkontinenzartikels (2) ausgehend von der Quermittelachse (30) bis zu einer die Hüftöffnung (18) begrenzenden Hüftöffnungskante (18') des Bauchabschnitts (4) mindestens 0,6, insbesondere mindestens 0,7 beträgt.

6. Inkontinenzartikel nach einem der vorstehenden Ansprüche, **dadurch gekennzeichnet, dass** der elastifizierte Abschnitt (94) des freien Längsrands (74) der Cuffelemente (68) in seiner Längserstreckung von der Quermittelachse (30) des Inkontinenzartikels (2) in Richtung Bauchabschnitt (4) um höchstens 10%, insbesondere höchstens 8% und insbesondere höchstens 6% größer ist bezogen auf die Längserstreckung von der Quermittelachse (30) des Inkontinenzartikels (2) in Richtung Rückenabschnitt (6), insbesondere dass der elastifizierte Abschnitt (94) des freien Längsrands (74) der Cuffelemente (68) in seiner Längserstreckung im Wesentlichen symmetrisch in Längsrichtung zur Quermittelachse angeordnet ist.

7. Inkontinenzartikel nach einem der vorstehenden Ansprüche, **dadurch gekennzeichnet, dass** die Cuffelemente (68) an die freien nicht elastifizierten Längsrandendabschnitte (93) in Längsrichtung anschließende fixierte Endrandabschnitte (98) aufweisen, mit denen die Cuffelemente (68) an der körperzugewandten Seite des Inkontinenzartikels (2) festgelegt sind, wobei die Cuffelemente (68) einwärts ihrer Cuffsockellinie (76) zu liegen kommen und insbesondere in den fixierten Endrandabschnitten (98) gefaltet eingebracht sind, insbesondere derart, dass der freie Längsrand (74) auswärts weist.

8. Inkontinenzartikel nach einem der vorstehenden Ansprüche, **dadurch gekennzeichnet, dass** das Verhältnis eines Abstands E zwischen den nach der Fixierung im fixierten Endrandabschnitt verbliebenen freien Kanten der Cuffelemente zu einem dem fixierten Endrandabschnitt nächstgelegenen maximalen Abstand D der Cuffsockellinien mindestens 0,3, weiter insbesondere mindestens 0,4, weiter insbesondere höchstens 0,7, weiter insbesondere höchstens 0,6, weiter insbesondere höchstens 0,5 beträgt.

9. Inkontinenzartikel nach einem der vorstehenden Ansprüche, **dadurch gekennzeichnet, dass** im Übergang eines vorderen Endes des elastifizierten Abschnitts (94) des freien Längsrands (74) zum nicht elastifizierten freien Längsrandendabschnitt (93) die Cuffelemente (68) eine Höhe und die Cuffsockellinien (76) einen Abstand zueinander aufweisen, wobei das Verhältnis der Höhe der Cuffelemente zum Abstand der Cuffsockellinien (76) mindestens 0,25, insbesondere mindestens 0,30, weiter insbesondere mindestens 0,35, weiter insbesondere höchstens 0,50, weiter insbesondere höchstens 0,45, weiter insbesondere höchstens 0,40 beträgt.

10. Inkontinenzartikel nach einem der vorstehenden Ansprüche, **dadurch gekennzeichnet, dass** im Übergang eines vorderen Endes des elastifizierten Abschnitts (94) des freien Längsrands (74) zum nicht elastifizierten freien Längsrandendabschnitt (93) die Cuffsockellinie (76) innerhalb eines Randes des Absorptionskörpers (7) angeordnet ist.

11. Inkontinenzartikel nach einem der vorstehenden Ansprüche, **dadurch gekennzeichnet, dass** das Verhältnis einer maximalen Höhe der Cuffelemente (68) ausgehend von der Cuffsockellinie (76) zum freien Längsrand (74) zur kleinsten Breite des Absorptionskörpers (7) mindestens 0,25, insbesondere mindestens 0,30, insbesondere mindestens 0,35 und insbesondere mindestens 0,40 sowie insbesondere höchstens 0,60, insbesondere höchstens 0,55 beträgt.

12. Inkontinenzartikel nach einem der vorstehenden Ansprüche, **dadurch gekennzeichnet, dass** ein Abstand M zwischen einer Längskante des Absorptionskörpers (7) an dessen kleinsten Breite und der davon auswärts angeordneten Cuffsockellinie (76) vorgesehen ist und das Verhältnis zwischen dem Abstand M und der kleinsten Breite des Absorptionskörper (7) mindestens 0,10, insbesondere mindestens 0,12, weiter insbesondere mindestens 0,14, weiter insbesondere höchstens 0,25, weiter insbesondere höchstens 0,22, weiter insbesondere höchstens 0,20 aufweist.

13. Inkontinenzartikel nach einem der vorstehenden Ansprüche, **dadurch gekennzeichnet, dass** im Wesentlichen eine gerade, insbesondere zur Längsmittelachse (44) parallel verlaufende Cuffsockellinie (76) im mittleren Bereich, insbesondere in Längsrichtung (9) innerhalb der schrittseitigen Enden der Seitennahtbereiche (14) vorgesehen ist.

14. Inkontinenzartikel nach einem der vorstehenden Ansprüche, **dadurch gekennzeichnet, dass** der Schrittabschnitt (8) außerhalb des Absorptionskörpers (7) entlang der Beinöffnungen (19) erstreckte Schrittelastifizierungsmittel (84) aufweist.

15. Inkontinenzartikel nach einem der vorstehenden Ansprüche, **dadurch gekennzeichnet, dass** in dem Bauchabschnitt (4) und/oder in dem Rückenabschnitt (6) erste Elastifizierungsmittel (28) vorgesehen sind, die sich in einem Abstand voneinander und im Wesentlichen in Quer- oder Hüftumfangsrichtung (16) erstrecken und so den Bauchabschnitt (4) und/oder den Rückenabschnitt (6) flächenhaft elastifizieren, und/oder dass der Bauchabschnitt (4) und/oder der Rückenabschnitt (6) in Quer- oder Hüftumfangsrichtung (16) elastische Flächenmaterialien umfasst oder wobei der Bauchabschnitt (4) und/oder der Rückenabschnitt (6) zumindest außerhalb des Absorptionskörpers (7) über die Längsrichtung (9) im Wesentlichen durchgehend flächenhaft querelastifiziert sind.

16. Inkontinenzartikel nach einem der vorstehenden Ansprüche, **dadurch gekennzeichnet, dass** der Anteil der Fläche des Schrittabschnitts (8) an der gesamten Fläche des Inkontinenzartikels 25 - 55 % beträgt, und dass der Schrittabschnitt (8) wenigstens 12 %, insbesondere 15-40%, insbesondere 15-35% der Fläche des Bauchabschnitts (4) und wenigstens 10 %, insbesondere 15 -35%, weiter insbesondere 15 - 30% der Fläche des Rückenabschnitts (6) überlappt.

17. Anordnung geschlechtsspezifischer Inkontinenzartikel in Höschenform, wobei die Anordnung umfasst:
Einen ersten Inkontinenzartikel (2) mit einer Längsmittelachse (44) und einer ersten Quermittelachse (30) für die Aufnahme von Körperausscheidungen, der angepasst ist von männlichen Personen getragen zu werden, wobei der Artikel einen in Längsrichtung voneinander beabstandeten vorderen Bauchabschnitt (4) und einen hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, umfasst, und einen einen ersten Absorptionskörper (7) aufweisenden Schrittabschnitt (8), der sich in einer Längsrichtung (9) zwischen Bauchabschnitt (4) und Rückenabschnitt (6) erstreckt und an den Bauchabschnitt und den Rückenabschnitt in einem jeweiligen Überlappungsbereich unlösbar angefügt ist, wobei der Schrittabschnitt (8), der Bauchabschnitt (4) und der Rückenabschnitt (6) gemeinsam Beinöffnungen (19) des Inkontinenzartikels begrenzen und mit eine seitliche Auslaufsperre bildenden und beidseits entlang der Längserstreckung des Absorptionskörpers verlaufenden Cuffelementen, die zumindest entlang einer Cuffsockellinie an der körperzugewandten Seite des Artikels festgelegt sind und einen ersten unbefestigten freien zumindest abschnittsweise in der Längsrichtung elastifizierten Längsrand aufweisen, mit dem sie sich von der körperzugewandten Seite des Artikels emporerheben können und so die jeweilige seitliche Auslaufsperre bilden, und mit einer ersten Quermittellinie des ersten Absorptionskörpers und
einen zweiten Inkontinenzartikel (2`) mit einer Längsmittelachse und einer zweiten Quermittelachse für die Aufnahme von Körperausscheidungen, der angepasst ist von weiblichen Personen getragen zu werden, wobei der Artikel einen in Längsrichtung voneinander beabstandeten vorderen Bauchabschnitt (4) und einen hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, umfasst, und einen einen zweiten Absorptionskörper (7`) aufweisenden Schrittabschnitt (8), der sich in einer Längsrichtung (9) zwischen Bauchabschnitt (4) und Rückenabschnitt (6) erstreckt und an den Bauchabschnitt und den Rückenabschnitt in einem jeweiligen Überlappungsbereich unlösbar angefügt ist wobei der Schrittabschnitt (8), der Bauchabschnitt (4) und der Rückenabschnitt (6) gemeinsam Beinöffnungen (19) des Inkontinenzartikels begrenzen und mit eine seitliche Auslaufsperre bildenden und beidseits entlang der Längserstreckung des Absorptionskörpers verlaufenden Cuffelementen, die zumindest entlang einer Cuffsockellinie an der körperzugewandten Seite des Artikels festgelegt sind und einen zweiten unbefestigten freien zumindest abschnittsweise in der Längsrichtung elastifizierten Längsrand aufweisen, mit dem sie sich von der körperzugewandten Seite des Artikels emporerheben können und so die jeweilige seitliche Auslaufsperre bilden, und mit einer zweiten Quermittellinie des zweiten Absorptionskörpers
**dadurch gekennzeichnet, dass** die erste Quermittellinie des ersten Absorptionskörpers bezüglich der ersten Quermittelachse des ersten Inkontinenzartikels in Richtung auf den Bauchabschnitt des Inkontinenzartikels im ersten Inkontinenzartikel weiter nach vorne verschoben angeordnet ist als die zweite Quermittellinie des zweiten Absorptionskörpers bezüglich der zweiten Quermittelachse des zweiten Inkontinenzartikels und dass der jeweilige unbefestigte freie Längsrand der beiden Cuffelemente des ersten Inkontinenzartikels zum vorderen Bauchabschnitt hin einen nicht elastifizierten freien Längsrandendabschnitt aufweist, wobei die Längserstreckung des nicht elastifizierten freien Längsrandendabschnitts C1 in Bezug auf eine Längserstreckung C2 des unbefestigten freien Längsrands der Cuffelemente in der sich von der Quermittelachse zum Bauchabschnitt erstreckenden Produkthälfte im ersten Inkontinenzartikel größer ist als im zweiten Inkontinenzartikel, dabei insbesondere um einen Faktor von mindestens 2, insbesondere mindestens 3, insbesondere mindestens 4, weiter insbesondere mindestens 5, weiter insbesondere um den Faktor von höchstens 10, weiter insbesondere höchstens 9 größer ist.

18. Anordnung nach Anspruch 17, **dadurch gekennzeichnet, dass** im ersten Inkontinenzartikel (2) die Längserstreckung C1 des nicht elastifizierten freien Längsrandendabschnitt (93) in Bezug auf eine Gesamtlängserstreckung C3 des unbefestigten freien Längsrands (74) der Cuffelemente (68) größer ist als im jeweils zweiten Inkontinenzartikel der nicht elastifizierte freie Längsrandendabschnitt (93), dabei insbesondere um den Faktor mindestens 2, weiter insbesondere mindestens 3, weiter insbesondere mindestens 4, weiter insbesondere mindestens 5, weiter insbesondere höchstens um den Faktor 12, insbesondere höchstens um den Faktor 10 größer ist.

19. Anordnung nach einem der vorangehenden Ansprüche 17 oder 18, **dadurch gekennzeichnet, dass** die Cuffelemente (68) des ersten Inkontinenzartikels (2) im Bereich der Quermittelachse (30) eine größere Höhe aufweisen als beim zweiten Inkontinenzartikel (2').

20. Anordnung nach einem der vorangehenden Ansprüche 17-19, **dadurch gekennzeichnet, dass** der erste Inkontinenzartikel nach einem der Ansprüche 1-16 ausgebildet ist.

## Claims

1. Incontinence article (2) in the form of briefs having a longitudinal central axis (44) and a transverse central axis (30) for receiving bodily excretions for the preferred use by men, comprising a front belly portion (4) and a rear back portion (6) which are spaced apart from one another in a longitudinal direction (9) and are connected to one another by the manufacturer at side seam regions (14) on both sides in order to form a belly and back band which is continuous in the transverse or hip-circumferential direction (16) and has a hip opening (18) which is closed in the hip-circumferential direction (16), and comprising a crotch portion (8) which has an absorption body (7) and extends in a longitudinal direction (9) between the belly portion (4) and the back portion (6) and is permanently attached to the belly portion (4) and the back portion (6) in a relevant overlap region, wherein the crotch portion (8), the belly portion (4) and the back portion (6) together delimit leg openings (19) of the incontinence article (2), and wherein the crotch portion (8) has cuff elements (68) which form a lateral leak barrier on both sides and extend along the longitudinal extension of the absorption body (7) on both sides, which cuff elements are secured at least along a cuff base line (76) to the side of the article (2) facing the body and have an unsecured free longitudinal edge (74) which is elasticized in the longitudinal direction at least in portions and by means of which the cuff elements (68) can be raised from the side of the incontinence article (2) facing the body and thus form the relevant lateral leak barrier, and wherein the absorption body (7) is arranged such that a transverse central line (43) of the absorption body (7) is arranged between the transverse central axis (30) of the incontinence article (2) and a hip opening edge (18') of the belly portion (4) that delimits the hip opening (18), **characterized in that** the relevant unsecured free longitudinal edge (74) of the two cuff elements (68) has a non-elasticized free longitudinal edge end portion (93) toward the front belly portion (4), the longitudinal extension Cl of which is at least 12%, in particular at least 15%, in particular at least 20%, and in particular at most 45%, in particular at most 40%, more particularly at most 35%, of a longitudinal extension C2 of the unsecured free longitudinal edge (74) of the cuff elements (68) in the product half extending from the transverse central axis (30) of the incontinence article (2) to the belly portion (4).

2. Incontinence article according to claim 1, **characterized in that** the relevant unsecured free longitudinal edge (74) of the two cuff elements (68) has a non-elasticized free longitudinal edge end portion (93) toward the front belly portion (4), the longitudinal extension Cl of which is at least 8%, in particular at least 10% and in particular at least 12%, and in particular at most 30%, in particular at most 28%, in particular at most 25% and in particular at most 23% of an overall longitudinal extension C3 of the unsecured free longitudinal edge (74) of the cuff elements (68).

3. Incontinence article according to claim 1 or 2, **characterized in that** the relevant cuff base line (76), in particular in the region of the non-elasticized free longitudinal edge end portion (93), has a cuff base line portion (76, 82) extending inward in the transverse direction (16), in particular in an arc shape, in the direction of a longitudinal central axis (44) of the incontinence article (2) and thus in the direction of the free longitudinal edge (76) of the relevant cuff element (68), wherein said cuff base line portion (76, 82) delimits, in the longitudinal direction, the region (74) of the cuff element (68) that can be raised.

4. Incontinence article according to any of the preceding claims, **characterized in that** the ratio of a length S1 of the absorption body (7) in front of the transverse central axis (30) of the incontinence article (2), in the direction of the belly portion (4), to a length S2 of the absorption body (7) behind the transverse central axis (30) of the incontinence article (2), in the direction of the back portion (6), is at least 1.2, preferably at least 1.3, and at most 2.0, in particular at most 1.8 and in particular at most 1.6.

5. Incontinence article according to any of the preceding claims, **characterized in that** the ratio of a length S1 of the absorption body (7) in front of the transverse central axis (30), in the direction of the belly portion (4), to a length L1 of the incontinence article (2) from the transverse central axis (30) to a hip opening edge (18') of the belly portion (4) that delimits the hip opening (18) is at least 0.6, in particular at least 0.7.

6. Incontinence article according to any of the preceding claims, **characterized in that** the elasticized portion(94) of the free longitudinal edge (74) of the cuff elements (68), in its longitudinal extension from the transverse central axis (30) of the incontinence article (2) in the direction of the belly portion (4), is at most 10%, in particular at most 8%, and in particular at most 6% greater than the longitudinal extension from the transverse central axis (30) of the incontinence article (2) in the direction of the back portion (6), in particular **in that** the longitudinal extension of the elasticized portion (94) of the free longitudinal edge (74) of the cuff elements (68) is arranged substantially symmetrically in the longitudinal direction with respect to the transverse central axis.

7. Incontinence article according to any of the preceding claims, **characterized in that** the cuff elements (68) have fixed end edge portions (98) which are connected to the free non-elasticized longitudinal edge end portions (93) in the longitudinal direction and by means of which the cuff elements (68) are secured to the side of the incontinence article (2) facing the body, wherein the cuff elements (68) come to lie inward with respect to their cuff base line (76) and are in particular inserted into the fixed end edge portions (98) in a folded manner, in particular such that the free longitudinal edge (74) points outward.

8. Incontinence article according to any of the preceding claims, **characterized in that** the ratio of a distance E between the free edges of the cuff elements remaining in the fixed end edge portion after the fixation to a maximum distance D between the cuff base lines closest to the fixed end edge portion is at least 0.3, more particularly at least 0.4, more particularly at most 0.7, more particularly at most 0.6, more particularly at most 0.5.

9. Incontinence article according to any of the preceding claims, **characterized in that** in the transition of a front end of the elasticized portion (94) of the free longitudinal edge (74) to the non-elasticized free longitudinal edge end portion (93), the cuff elements (68) have a height and the cuff base lines (76) are at distance from one another, wherein the ratio of the height of the cuff elements to the distance between the cuff lines (76) is at least 0.25, in particular at least 0.30, more particularly at least 0.35, more particularly at most 0.50, more particularly at most 0.45, more particularly at most 0.40.

10. Incontinence article according to any of the preceding claims, **characterized in that** in the transition of a front end of the elasticized portion (94) of the free longitudinal edge (74) to the non-elasticized free longitudinal edge end portion (93), the cuff base line (76) is arranged within an edge of the absorption body (7) .

11. Incontinence article according to any of the preceding claims, **characterized in that** the ratio of a maximum height of the cuff elements (68), from the cuff base line (76) to the free longitudinal edge (74), to the smallest width of the absorption body (7) is at least 0.25, in particular at least 0.30, in particular at least 0.35 and in particular at least 0.40, and in particular at most 0.60, in particular at most 0.55.

12. Incontinence article according to any of the preceding claims, **characterized in that** there is a distance M between a longitudinal edge of the absorption body (7) at its smallest width and the cuff base line (76) arranged outwardly therefrom, and the ratio between the distance M and the smallest width of the absorption body (7) is at least 0.10, in particular at least 0.12, more particularly at least 0.14, more particularly at most 0.25, more particularly at most 0.22, more particularly at most 0.20.

13. Incontinence article according to any of the preceding claims, **characterized in that** a straight cuff base line (76), in particular extending in parallel with the longitudinal central axis (44), is substantially provided in the central region, in particular in the longitudinal direction (9) within the crotch-side ends of the lateral seam regions (14).

14. Incontinence article according to any of the preceding claims, **characterized in that** the crotch portion (8) has crotch-elasticizing means (84) extending along the leg openings (19) outside the absorption body (7).

15. Incontinence article according to any of the preceding claims, **characterized in that** first elasticizing means (28) are provided in the belly portion (4) and/or in the back portion (6), which first elasticizing means extend at a distance from one another and substantially in the transverse or hip-circumferential direction (16) and thus elasticize the belly portion (4) and/or the back portion (6) in a planar manner, and/or **in that** the belly portion (4) and/or the back portion (6) comprises elastic planar materials in the transverse or hip-circumferential direction (16), or wherein the belly portion (4) and/or the back portion (6), at least outside the absorption body (7), are transversely elasticized substantially continuously over the longitudinal direction (9) in a planar manner.

16. Incontinence article according to any of the preceding claims, **characterized in that** the proportion of the area of the crotch portion (8) of the entire area of the incontinence article is 25-55%, and **in that** the crotch portion (8) overlaps with at least 12%, in particular 15-40%, in particular 15-35% of the area of the belly portion (4) and with at least 10%, in particular 15-35%, more particularly 15-30% of the area of the back portion (6).

17. Arrangement of a gender-specific incontinence article in the form of briefs, the arrangement comprising:
a first incontinence article (2) having a longitudinal central axis (44) and a first transverse central axis (30) for receiving bodily excretions, which is adapted to be worn by male persons, wherein the article comprises a front belly portion (4) and a rear back portion (6) which are spaced apart from one another in the longitudinal direction and are connected to one another by the manufacturer at side seam regions (14) on both sides in order to form a belly and back band which is continuous in the transverse or hip-circumferential direction (16) and has a hip opening (18) which is closed in the hip-circumferential direction, and a crotch portion (8) which has a first absorption body (7) and extends in a longitudinal direction (9) between the belly portion (4) and the back portion (6), and which is permanently attached to the belly portion and the back portion in a relevant overlap region, wherein the crotch portion (8), the belly portion (4) and the back portion (6) together delimit leg openings (19) of the incontinence article, and comprising cuff elements which form a lateral leak barrier on both sides and extend along the longitudinal extension of the absorption body on both sides, which cuff elements are secured at least along a cuff base line to the side of the article facing the body and have a first unsecured free longitudinal edge which is elasticized in the longitudinal direction at least in portions and by means of which the cuff elements can be raised from the side of the article facing the body and thus form the relevant leak barrier, and comprising a first transverse central line of the first absorption body, and
a second incontinence article (2') having a longitudinal central axis and a second transverse central axis for receiving bodily excretions, which is adapted to be worn by female persons, wherein the article comprises a front belly portion (4) and a rear back portion (6) which are spaced apart from one another in the longitudinal direction and are connected to one another by the manufacturer at side seam regions (14) on both sides in order to form a belly and back band which is continuous in the transverse or hip-circumferential direction (16) and has a hip opening (18) which is closed in the hip-circumferential direction, and a crotch portion (8) which has a second absorption body (7') and extends in a longitudinal direction (9) between the belly portion (4) and the back portion (6), and which is permanently attached to the belly portion and the back portion in a relevant overlap region, wherein the crotch portion (8), the belly portion (4) and the back portion (6) together delimit leg openings (19) of the incontinence article, and comprising cuff elements which form a lateral leak barrier on both sides and extend along the longitudinal extension of the absorption body on both sides, which cuff elements are secured at least along a cuff base line to the side of the article facing the body and have a second unsecured free longitudinal edge which is elasticized in the longitudinal direction at least in portions and by means of which the cuff elements can be raised from the side of the article facing the body and thus form the relevant leak barrier, and comprising a second transverse central line of the second absorption body,
**characterized in that,** in the first incontinence article, the first transverse central line of the first absorption body is arranged further forward in the direction of the belly portion of the incontinence article with respect to the first transverse central axis of the first incontinence article than the second transverse central line of the second absorption body is arranged with respect to the second transverse central axis of the second incontinence article, and **in that** the relevant unsecured free longitudinal edge of the two cuff elements of the first incontinence article has a non-elasticized free longitudinal edge end portion toward the front belly portion, wherein the longitudinal extension of the non-elasticized free longitudinal edge portion Cl, with respect to a longitudinal extension C2 of the unsecured free longitudinal edge of the cuff elements in the product half extending from the transverse central axis to the belly portion, is greater in the first incontinence article than in the second incontinence article, in particular by a factor of at least 2, in particular at least 3, in particular at least 4, more particularly at least 5, more particularly by a factor of at most 10, more particularly at most 9.

18. Arrangement according to claim 17, **characterized in that**, in the first incontinence article (2), the longitudinal extension Cl of the non-elasticized free longitudinal edge portion (93) with respect to an overall longitudinal extension C3 of the unsecured free longitudinal edge (74) of the cuff elements (68) is greater than the non-elasticized free longitudinal edge portion (93) in the relevant second incontinence article, in particular by a factor of at least 2, more particularly at least 3, more particularly at least 4, more particularly at least 5, more particularly at most by a factor of 12, in particular at most by a factor of 10.

19. Arrangement according to one of the preceding claims 17 or 18, **characterized in that** the cuff elements (68) of the first incontinence article (2) have a greater height in the region of the transverse central axis (30) than in the second incontinence article (2').

20. Arrangement according to any of the preceding claims 17-19, **characterized in that** the first incontinence article is designed according to any of claims 1-16.

## Revendications

1. Article d'incontinence (2) sous forme de culotte avec un axe médian longitudinal (44) et un axe médian transversal (30) pour la réception d'excrétions corporelles destiné à être utilisé de préférence par des hommes, avec une partie ventrale (4) avant et une partie dorsale (6) arrière espacées l'une de l'autre dans une direction longitudinale (9), qui pour la formation d'une bande ventrale et dorsale continue dans la direction transversale ou du tour des hanches (16) avec une ouverture pour les hanches (18) -fermée dans la direction du tour des hanches (16) sur les zones de couture latérale (14) bilatérales sont reliées l'une à l'autre chez le fabricant, et avec une partie d'entrejambe (8) présentant un corps absorbant (7), qui s'étend dans une direction longitudinale (9) entre la partie ventrale (4) et la partie dorsale (6) et est jointe de manière indétachable à la partie ventrale (4) et à la partie dorsale (6) dans une zone de chevauchement respective, dans lequel la partie d'entrejambe (8), la partie ventrale (4) et la partie dorsale (6) délimitent ensemble les ouvertures pour les jambes (19) de l'article d'incontinence (2) et dans lequel la partie d'entrejambe (8) présente des deux côtés des éléments de revers (68) formant une barrière anti-fuites latérale et s'étendant des deux côtés le long de l'étendue longitudinale du corps absorbant (7), qui sont immobilisés au moins le long d'une ligne de base de revers (76) sur la face de l'article (2) tournée vers le corps et présentent un bord longitudinal libre non fixé (74), qui est élastifié au moins sur certaines parties dans la direction longitudinale, avec lequel les éléments de revers (68) peuvent se soulever de la face de l'article d'incontinence (2) tournée vers le corps et forment ainsi la barrière anti-fuites latérale respective et dans lequel le corps absorbant (7) est disposé de sorte qu'une ligne médiane transversale (43) du corps absorbant (7) est disposée entre l'axe médian transversal (30) de l'article d'incontinence (2) et une arête d'ouverture pour les hanches (18') de la partie ventrale (4) délimitant l'ouverture pour les hanches (18), **caractérisé en ce que** le bord longitudinal libre non fixé (74) respectif des deux éléments de revers (68) présente en direction de la partie ventrale avant (4) une partie d'extrémité de bord longitudinal libre non élastifiée (93), dont l'étendue longitudinale C1 atteint au moins 12%, en particulier au moins 15%, en particulier au moins 20% et en particulier au plus 45%, en particulier au plus 40%, plus particulièrement au plus 35% d'une étendue longitudinale C2 du bord longitudinal libre non fixé (74) des éléments de revers (68) dans la moitié du produit s'étendant à partir de l'axe médian transversal (30) de l'article d'incontinence (2) vers la partie ventrale (4).

2. Article d'incontinence selon la revendication 1, **caractérisé en ce que** le bord longitudinal libre non fixé (74) respectif des deux éléments de revers (68) présente en direction de la partie ventrale (4) avant une partie d'extrémité de bord longitudinal libre non élastifiée (93), dont l'étendue longitudinale C1 atteint au moins 8%, en particulier au moins 10% et en particulier au moins 12% ainsi qu'en particulier au plus 30%, en particulier au plus 28%, en particulier au plus 25% et en particulier au plus 23% d'une étendue longitudinale totale C3 du bord longitudinal libre non fixé (74) des éléments de revers (68).

3. Article d'incontinence selon la revendication 1 ou 2, **caractérisé en ce que** la ligne de base de revers (76) respective présente, en particulier dans la zone de la partie d'extrémité de bord longitudinal libre non élastifiée (93), une partie de ligne de base de revers (76, 82) s'étendant dans la direction transversale (16), en particulier de manière arquée, vers l'intérieur en direction d'un axe médian longitudinal (44) de l'article d'incontinence (2) et donc en direction du bord longitudinal libre (76) de l'élément de revers (68) respectif, dans lequel cette partie de ligne de base de revers (76, 82) délimite dans la direction longitudinale la zone (74) pouvant être soulevée de l'élément de revers (68).

4. Article d'incontinence selon l'une quelconque des revendications précédentes, **caractérisé en ce que** le rapport d'une longueur S1 du corps absorbant (7) devant l'axe médian transversal (30) de l'article d'incontinence (2) en direction de la partie ventrale (4) à une longueur S2 du corps absorbant (7) derrière l'axe médian transversal (30) de l'article d'incontinence (2) en direction de la partie dorsale (6) atteint au moins 1,2, de préférence au moins 1,3 et au plus 2,0, en particulier au plus 1,8 et en particulier au plus 1,6.

5. Article d'incontinence selon l'une quelconque des revendications précédentes, **caractérisé en ce que** le rapport d'une longueur S1 du corps absorbant (7) devant l'axe médian transversal (30) en direction de la partie ventrale (4) à une longueur L1 de l'article d'incontinence (2) à partir de l'axe médian transversal (30) jusqu'à une arête d'ouverture pour les hanches (18') de la partie ventrale (4) délimitant l'ouverture pour les hanches (18) atteint au moins 0,6, en particulier au moins 0,7.

6. Article d'incontinence selon l'une quelconque des revendications précédentes, **caractérisé en ce que** la partie élastifiée (94) du bord longitudinal libre (74) des éléments de revers (68) dans son étendue longitudinale à partir de l'axe médian transversal (30) de l'article d'incontinence (2) en direction de la partie ventrale (4) est plus grande d'au plus 10%, en particulier d'au plus 8% et en particulier d'au plus 6% par rapport à l'étendue longitudinale à partir de l'axe médian transversal (30) de l'article d'incontinence (2) en direction de la partie dorsale (6), en particulier que la partie élastifiée (94) du bord longitudinal libre (74) des éléments de revers (68) dans son étendue longitudinale est disposée de manière sensiblement symétrique dans la direction longitudinale par rapport à l'axe médian transversal.

7. Article d'incontinence selon l'une quelconque des revendications précédentes, **caractérisé en ce que** les éléments de revers (68) présentent des parties de bord d'extrémité (98) fixées se raccordant dans la direction longitudinale aux parties d'extrémité de bord longitudinal libres non élastifiées (93), avec lesquelles les éléments de revers (68) sont immobilisés sur la face de l'article d'incontinence (2) tournée vers le corps, dans lequel les éléments de revers (68) viennent se situer en dedans de leur ligne de base de revers (76) et en particulier sont introduits de manière pliée dans les parties de bord d'extrémité fixées (98), en particulier de telle sorte que le bord longitudinal libre (74) soit dirigé vers l'extérieur.

8. Article d'incontinence selon l'une quelconque des revendications précédentes, **caractérisé en ce que** le rapport d'une distance E entre les arêtes libres des éléments de revers restant après la fixation dans la partie de bord d'extrémité fixée à une distance maximale D des lignes de base de revers la plus proche de la partie de bord d'extrémité fixée atteint au moins 0,3, plus particulièrement au moins 0,4, plus particulièrement au plus 0,7, plus particulièrement au plus 0,6, plus particulièrement au plus 0,5.

9. Article d'incontinence selon l'une quelconque des revendications précédentes, **caractérisé en ce que** dans la transition d'une extrémité avant de la partie élastifiée (94) du bord longitudinal libre (74) vers la partie d'extrémité de bord longitudinal non élastifiée (93) les éléments de revers (68) présentent une hauteur et les lignes de base de revers (76) une distance les uns par rapport aux autres, dans lequel le rapport de la hauteur des éléments de revers à la distance des lignes de base de revers (76) atteint au moins 0,25, en particulier au moins 0,30, plus particulièrement au moins 0,35, plus particulièrement au plus 0,50, plus particulièrement au plus 0,45, plus particulièrement au plus 0,40.

10. Article d'incontinence selon l'une quelconque des revendications précédentes, **caractérisé en ce que** dans la transition d'une extrémité avant de la partie élastifiée (94) du bord longitudinal libre (74) vers la partie d'extrémité de bord longitudinal libre non élastifiée (93) la ligne de base de revers (76) est disposée à l'intérieur d'un bord du corps absorbant (7) .

11. Article d'incontinence selon l'une quelconque des revendications précédentes, **caractérisé en ce que** le rapport d'une hauteur maximale des éléments de revers (68) à partir de la ligne de base de revers (76) vers le bord longitudinal libre (74) à la plus petite largeur du corps absorbant (7) atteint au moins 0,25, en particulier au moins 0,30, en particulier au moins 0,35 et en particulier au moins 0,40 ainsi qu'en particulier au plus 0,60, en particulier au plus 0,55.

12. Article d'incontinence selon l'une quelconque des revendications précédentes, **caractérisé en ce qu'**une distance M entre une arête longitudinale du corps absorbant (7) sur la plus petite largeur de celui-ci et la ligne de base de revers (76) disposée en dehors de celle-ci est prévue et le rapport entre la distance M et la plus petite largeur du corps absorbant (7) présente au moins 0,10, en particulier au moins 0,12, plus particulièrement au moins 0,14, plus particulièrement au plus 0,25, plus particulièrement au plus 0,22, plus particulièrement au plus 0,20.

13. Article d'incontinence selon l'une quelconque des revendications précédentes, **caractérisé en ce que** sensiblement une ligne de base de revers (76) droite, en particulier s'étendant parallèlement à l'axe médian longitudinal (44), est prévue dans la zone centrale, en particulier dans la direction longitudinale (9) à l'intérieur des extrémités côté entrejambe des zones de couture latérale (14).

14. Article d'incontinence selon l'une quelconque des revendications précédentes, **caractérisé en ce que** la partie d'entrejambe (8) présente à l'extérieur du corps absorbant (7) des moyens d'élastification à l'entrejambe (84) étendus le long des ouvertures pour les jambes (19) .

15. Article d'incontinence selon l'une quelconque des revendications précédentes, **caractérisé en ce que** des premiers moyens d'élastification (28), qui s'étendent à distance les uns des autres et sensiblement dans la direction transversale ou du tour des hanches (16) et ainsi élastifient la partie ventrale (4) et/ou la partie dorsale (6) sur toute la surface, sont prévus dans la partie ventrale (4) et/ou dans la partie dorsale (6) et/ou que la partie ventrale (4) et/ou la partie dorsale (6) comprend dans la direction transversale ou du tour des hanches (16) des matériaux en feuille élastiques ou dans lequel la partie ventrale (4) et/ou la partie dorsale (6) sont élastifiées transversalement sur toute la surface sensiblement en continu au moins à l'extérieur du corps absorbant (7) sur toute l'étendue de la direction longitudinale (9).

16. Article d'incontinence selon l'une quelconque des revendications précédentes, **caractérisé en ce que** le pourcentage de la surface de la partie d'entrejambe (8) sur toute la surface de l'article d'incontinence atteint 25-55 %, et que la partie d'entrejambe (8) chevauche au moins 12 %, en particulier 15-40 %, en particulier 15-35 % de la surface de la partie ventrale (4) et au moins 10 %, en particulier 15-35 %, plus particulièrement 15-30 % de la surface de la partie dorsale (6).

17. Ensemble d'articles d'incontinence spécifiques au sexe sous forme de culottes, dans lequel l'ensemble comprend :
un premier article d'incontinence (2) avec un axe médian longitudinal (44) et un premier axe médian transversal (30) pour la réception d'excrétions corporelles, qui est adapté à être porté par des personnes masculines, dans lequel l'article comprend une partie ventrale (4) avant et une partie dorsale arrière (6) espacées l'une de l'autre dans la direction longitudinale, qui pour la formation d'une bande ventrale et dorsale continue dans la direction transversale ou du tour des hanches (16) avec une ouverture pour les hanches (18) ·fermée dans la direction du tour des hanches (16) sur les zones de couture latérale (14) bilatérales sont reliées l'une à l'autre chez le fabricant, et une partie d'entrejambe (8) présentant un premier corps absorbant (7), qui s'étend dans une direction longitudinale (9) entre la partie ventrale (4) et la partie dorsale (6) et est jointe de manière indétachable à la partie ventrale et à la partie dorsale dans une zone de chevauchement respective, dans lequel la partie d'entrejambe (8), la partie ventrale (4) et la partie dorsale (6) délimitent ensemble les ouvertures pour les jambes (19) de l'article d'incontinence et avec des éléments de revers formant une barrière antifuites latérale et s'étendant des deux côtés le long de l'étendue longitudinale du corps absorbant, qui sont immobilisés au moins le long d'une ligne de base de revers sur la face de l'article tournée vers le corps et présentent un premier bord longitudinal libre non fixé élastifié au moins sur certaines parties dans la direction longitudinale, avec lequel ils peuvent se soulever de la face de l'article tournée vers le corps et forment ainsi la barrière anti-fuites latérale respective, et avec une première ligne médiane transversale du premier corps absorbant et
un deuxième article d'incontinence (2') avec un axe médian longitudinal et un deuxième axe médian transversal pour la réception d'excrétions corporelles, qui est adapté à être porté par des personnes féminines, dans lequel l'article comprend une partie ventrale (4) avant et une partie dorsale (6) arrière espacées l'une de l'autre dans la direction longitudinale, qui pour la formation d'une bande ventrale et dorsale continue dans la direction transversale ou du tour des hanches (16) avec une ouverture pour les hanches (18) -fermée dans la direction du tour des hanches (16) sur les zones de couture latérale (14) bilatérales sont reliées l'une à l'autre chez le fabricant, et une partie d'entrejambe (8) présentant un deuxième corps absorbant (7'), qui s'étend dans une direction longitudinale (9) entre la partie ventrale (4) et la partie dorsale (6) et est jointe de manière indétachable à la partie ventrale et à la partie dorsale dans une zone de chevauchement respective, dans lequel la partie d'entrejambe (8), la partie ventrale (4) et la partie dorsale (6) délimitent ensemble les ouvertures pour les jambes (19) de l'article d'incontinence et avec des éléments de revers formant une barrière anti-fuites latérale et s'étendant des deux côtés le long de l'étendue longitudinale du corps absorbant, qui sont fixés au moins le long d'une ligne de base de revers sur la face de l'article tournée vers le corps et présentent un deuxième bord longitudinal libre non fixé élastifié au moins sur certaines parties dans la direction longitudinale, avec lequel ils peuvent se soulever de la face de l'article tournée vers le corps et forment ainsi la barrière anti-fuites latérale respective, et avec une deuxième ligne médiane transversale du deuxième corps absorbant
**caractérisé en ce que** la première ligne médiane transversale du premier corps absorbant par rapport au premier axe médian transversal du premier article d'incontinence en direction de la partie ventrale de l'article d'incontinence dans le premier article d'incontinence est disposée de manière glissée davantage vers l'avant que la deuxième ligne médiane transversale du deuxième corps absorbant par rapport au deuxième axe médian transversal du deuxième article d'incontinence et que le bord longitudinal libre non fixé respectif des deux éléments de revers du premier article d'incontinence présente en direction de la partie ventrale avant une partie d'extrémité de bord longitudinal libre non élastifiée, dans lequel l'étendue longitudinale de la partie d'extrémité de bord longitudinal libre non élastifiée C1 par rapport à une étendue longitudinale C2 du bord longitudinal libre non fixé des éléments de revers dans la moitié du produit s'étendant à partir de l'axe médian transversal vers la partie ventrale dans le premier article d'incontinence est plus grande que dans le deuxième article d'incontinence, ce faisant est plus grande en particulier d'un facteur d'au moins 2, en particulier d'au moins 3, en particulier d'au moins 4, plus particulièrement d'au moins 5, plus particulièrement du facteur d'au plus 10, plus particulièrement d'au plus 9.

18. Ensemble selon la revendication 17, **caractérisé en ce que** dans le premier article d'incontinence (2) l'étendue longitudinale C1 de la partie d'extrémité de bord longitudinal libre non élastifiée (93) par rapport à une étendue longitudinale totale C3 du bord longitudinal libre non fixé (74) des éléments de revers (68) est plus grande que dans le respectivement deuxième article d'incontinence de la partie d'extrémité de bord longitudinal libre non élastifiée (93), ce faisant est plus grande en particulier du facteur d'au moins 2, plus particulièrement d'au moins 3, plus particulièrement d'au moins 4, plus particulièrement d'au moins 5, plus particulièrement d'au plus du facteur 12, en particulier au plus du facteur 10.

19. Ensemble selon l'une quelconque des revendications précédentes 17 ou 18, **caractérisé en ce que** les éléments de revers (68) du premier article d'incontinence (2) dans la zone de l'axe médian transversal (30) présentent une plus grande hauteur que pour le deuxième article d'incontinence (2').

20. Ensemble selon l'une quelconque des revendications précédentes 17-19, **caractérisé en ce que** le premier article d'incontinence est réalisé selon l'une quelconque des revendications 1-16.
